# EUROPEAN PATENT SPECIFICATION

(11) **EP 0 636 171 B1**
(45) Date of publication and mention of the grant of the patent: **07.06.2000**
(21) Application number: 93908527.0
(22) Date of filing: 24.03.1993
(51) Int. Cl.: C12N 5/00, C07K 16/30, A61K 39/395

(54) **METHODS OF USING ANTIBODIES AGAINST HUMAN CHORIONIC GONADOTROPIN-RELATED DETERMINANTS TO LYSE MALIGNANT CELLS**
METHODEN DER ANWENDUNG VON ANTIKÖRPERN GEGEN HUMANE CHORIONIC GONADOTROPIN VERWANDTE DETERMINANTEN ZUR LYSE VON KRANKEN ZELLEN
PROCEDES RECOURANT A DES ANTICORPS CONTRE DES DETERMINANTS LIES A LA GONADOTROPINE CHORIONIQUE HUMAINE AFIN DE LYSER DES CELLULES MALIGNES

(30) Priority: 24.03.1992 US 856455
(43) Date of publication of application: 01.02.1995
(73) Proprietor: The Trustees of Columbia University in the City of New York, New York, NY 10027-6699 (US); ALLEGHENY-SINGER RESEARCH INSTITUTE, Pittsburgh, PA 15212-9986 (US)
(72) Inventor: ACEVEDO, Hernan, Pittsburgh, PA 15237 (US); KRICHEVSKY, Alexander, New York, NY 10040 (US)
(74) Representative: Van Someren, Petronella F. H. M.
(86) International application number: US9302710
(87) International publication number: WO9319167

(56) References cited:
- EP-A- 0 234 122
- EP-A- 0 421 392
- BURCHIEL S.W. ET AL. 'The Radio Immunochemical Detection of Cancer ' 1982 , BURCHIEL S.W., RHODES B.A., FRIEDMAN B, EDS. , NEW YORK Chapter 10 SCOTT W. BURCHIEL ET AL.: " Expression and detection of hCG in human malignancy." * page 92, right column, paragraph 2 - page 93, right column, paragraph 2 * * page 96, left column, last paragraph - right column, paragraph 2 *
- ENDOCRINOLOGY, vol. 128, no. 3, March 1991 pages 1255-1264, ALEXANDER KRICHEVSKY ET AL. 'Development and characterization of a new, highly specific antibody to the human chorionic gonadotropin-beta fragment'
- FUDENBERG et al. (Eds), "Basic and Clinical Immunology", published 1976 by LANGE Medical Publications (Los Altos, California), pages 225-241, see pages 228 and 231 in particular.
- Endocrinology, Volume 123, No. 1, issued July 1988, A. KRICHEVSKY et al., "Preparation and Characterization of Antibodies to the Urinary Fragment of the Human Chorionic Gonadotropin Beta-Subunit", pages 584-593, see Table I in particular.
- Cancer Detection and Prevention Supplement, Volume 1, issued 1987, R.B. RAIKOW et al., "Flow Cytofluorometric Analysis of Choriogonadotropin-Like Material on the Surface of Human and Mouse Malignant Cells", pages 173-181, see page 173 in particular.
- Cancer 1992 vol 69,no7 pages 1818-1828

## Description

### Background of the Invention

Throughout this application various publications are referenced by Arabic numerals. Full citations for these references may be found at the end of the specification immediately preceding the claims.

The hormone of pregnancy, human chorionic gonadotropin (hCG), is a dimeric sialoglycoprotein composed of two noncovalently linked dissimilar subunits known as α and β. The single α-subunit (hCGα) gene is located in chromosome 6 (1-3). A cluster of at least six genes (4, 5) located in chromosome 19 (3) encode individually for the β subunit (hCGβ). Within species, hCGα is identical to the α-subunit of the three other members that constitute the family of glycoprotein hormones arising from the anterior pituitary, that is lutropin, follitropin and thyrotropin. The unique β-subunits determine the biological specificity for each one of these hormones (6). Under normal nonpregnant conditions, only sperm cells in male humans (7, 8) and cells from some fetal tissues (9) synthesize the subunits de novo.

Expression of the subunits, as well as translatable levels of hCGβ mRNA, have been detected early after fertilization (10-13).

The dimer, which is the intact or whole compound (hCG-holo), is the only bioactive molecule. Its formation depends not only on the different factors regulating the expression of the α-subunit and β-subunit genes (14, 15) but also on the synthesis of competent subunits (16-19). Therefore, in cells that synthesize hCG, free subunits can also be found. Another important chemical characteristic of these highly glycosylated proteins (the carbohydrate component of intact hCG comprises approximately one third of its 37 KD molecular weight) is their high degree of sialylation. Thus, hCGβ has up to 12 sialic acid residues, 8 of them in the carboxy terminal peptide (CTP) (residues 111 to 145), and hCGα has 4, resulting in molecules having a high negative charge.

In addition, hCG-holo and its subunits are present in the cell in two pools, a secretory pool (hydrophilic, also known as the soluble pool) and a membrane pool, also described as the insoluble pool. In the latter pool, the dimer and its subunits become an intrinsic part of the cell membrane and cannot be extracted unless the membranes are destroyed by sonication and treated with detergents to solubilize the material--hence the name insoluble pool (20).

During the last 5 years, several pieces of information pertaining to the chemistry and biology of hCG have been published. The first was the elucidation of the structure of the urinary hCGβ core fragment, a dimer formed by peptides lacking 72 of the 145 amino acid residues of hCGβ, including the highly glycosylated (and sialylated) CTP of hCGβ (hCGβ-CTP). The latter does not combine with the α-subunit, cross-reacts with practically all hCGβ antisera and most monoclonal antibodies (MoAb) (21, 22), and contaminates in various degrees all hCG standard preparations. This is one of the main reasons for the heterogeneity and differences between immunoreactivity and bioactivity of these preparations (23).

The second was the finding, using high-resolution high-performance liquid chromatography fingerprinting of purified hCG together with analysis of selected peptides by amino acid sequencing and mass spectroscopy, that oxidation, not carbohydrate variability, is another cause of the chromatographic heterogeneity of the hCG isolated from pregnancy urine, the source of all existing hCG preparations (24).

The third was a recent report that this glycopeptide may circulate in blood associated with other macromolecules that mask its epitopes to existing hCGβ antibodies (25).

This new information (added to the potential for producing alterations in the biophysical and physiological characteristics of the cell membrane as a result of the existence of the membrane-associated hCG pool) and the reports that the metastatic potential of cultured murine tumor cell lines correlated with the amount of cell membrane sialylation (26) and increased negative charge (27) created the need for a practical quantitative method sensitive enough to measure this pool.

The possibility of using flow cytometry for the analysis of membrane-associated hCG, it subunits, and fragments was demonstrated in 1982 (28). This report was followed by feasibility studies on the use of a Coulter Epics-C flow cytometer (Hialeah, FL) for determining chorionic gonadotropin-like material on the cell surface of malignant cells (29). However, there was still absent any method of accurately quantitatively analyzing hGC, its subunits and fragments as membrane-associated material.

Such a method that was used in the research that led to the invention is described in the Examples.

In EP-A-234 122 it is described that antibodies which are directed to glycolipid antigens at the surface of tumor cells and which belong to a class of immunoglobulin capable of activating serum complement and/or mediating antibody dependent cellular cytotoxicity by human effector cells may be used to lyse tumor cells expressing these glycolipid antigens.

It is the object of the invention to provide alternative antibodies for the purpose of treating malignant forms of cancer.

### Summary of the Invention

This invention provides a method of lysing malignant cells, which comprises contacting the malignant cells with an antibody which specifically reacts with an epitope present on the beta subunit of human chorionic gonadotropin and/or beta subunit fragment, under conditions much that the malignant cells lyse.

### Detailed Description of the Invention

Specifically, this invention provides a method of lysing malignant cells, which comprises contacting the malignant cells with an antibody which specifically reacts with an epitope present on the beta subunit of human chorionic gonadotropin and/or beta subunit fragment, under conditions such that the malignant cells lyse.

As used in the subject invention, the malignant cells to be lysed include, but are in no way limited to, sarcoma cells, lymphoma cells and retinoblastoma cells.

As used in the subject invention, the term "antibody" includes, but is not limited to, both naturally occurring and non-naturally occurring antibodies. Specifically, the term "antibody" includes polyclonal and monoclonal antibodies, and binding fragments thereof. Furthermore, the term "antibody" includes chimeric antibodies and wholely synthetic antibodies, and fragments thereof.

The antibody may be a monoclonal antibody. In one embodiment of the invention, the monoclonal antibody is the monoclonal antibody designated B201. In another embodiment of the invention, the monoclonal antibody is designated B204.

The method of the subject invention may be used to treat a patient suffering from a malignant form of cancer. Specifically, treating the patient may comprise administering to the patient an amount of antibody which specifically reacts with an epitope present on the beta subunit of human chorionic gonadotropin and/or beta effective to lyse malignant cells in the patient's body.

The administration may comprise intravenous, subcutaneous, or intraperitoneal administration, or any other route of administration well known to those skilled in the art. Furthermore, the antibody may be administered using a pharmaceutical carrier well known to those skilled in the art.

This invention will be better understood by reference to the Experimental Details which follow, but those skilled in the art will readily appreciate that the specific experiments detailed are only illustrative of the invention as described more fully in the claims which follow thereafter. The flow cytometry method that is used in the research that led to the invention is described first.

### Experimental Details

### I. Flow Cytometry Method For The Analysis Of Membrane-Associated Human Chorionic Gonadotropin, Its Subunits And Fragments on Human Cancer Cells

### Introduction

The combination of flow cytometry and MoAb binding to different epitopes located in different parts of the hCG molecule has produced a method that has three advantages: 1) the use of live cells, 2) a high degree of sensitivity that can detect as few as 10³ molecules of fluorochrome per cell (30), and 3) a good supply of standardized reagents. Presented here is a detailed description of this new method, its reliability and reproducibility, and some examples of its application to the measurement of membrane-associated hCG in cultured human malignant cells.

### Materials and Methods

### Cell Cultures

HeLa cells (adenocarcinoma of uterine cervix) and two of its variants, HeLa 229, and HeLa S3 were obtained from the American Type Culture Collection (ATCC; ATCC CCL 2, CCL 2.1, and CCL 2.2, respectively). The origin, characteristics and references of these cell lines are given in the ATCC catalog (31). JEG-3 cells, a clone 3 of the choriocarcinoma BeWo cells (32-34) are available from ATCC (ATCC HTB 36). All cells were grown in a humidified incubator at 37°C and 5% CO₂ in RPMI-1640 medium (JRH Biosciences, Lenexa, KS) with 10% defined iron-supplemented bovine calf serum (HyClone, Logan, UT), 2 mmol/1 L-glutamine (Fisher Scientific, Fairlawn, NJ), 1 mmol/1 sodium pyruvate (JRH Biosciences), and 20 mg/1 of gentamicin sulfate (Sigma, St. Louis, MO). In one experiment, JEG-3 cells were grown in minimum essential medium (MEM), alpha modification (Sigma) with 4% fetal bovine serum (HyClone) and 20 mg/1 of gentamicin sulfate. The cells were cultured in 25-cm² and 75-cm² tissue culture flasks. For passage or analysis, the cells were detached using trypsin 0.25% with ethylenediamine tetracetic acid 0.1% (JRH Biosciences) or a cell scraper (Costar, Cambridge, MA). All cells were harvested at confluence for analysis. A hemacytometer was used to count the cells for all experiments, and the viability was determined by dye exclusion with trypan blue.

### Cell Controls

Because ectopic de novo synthesis, cell membrane localization, and secretion of subunits and bioactive hCG by HeLa cells has been well established (35-37), the ATCC CCL 2 HeLa cell line was selected for use as a cell control for membrane-associated hCG, its subunits, and fragments and for overall quality control of the method.

### Antibodies

The development of the method using the indirect or double-antibody technique required the testing of numerous antibodies to select the most appropriate ones to be used as first (or primary) antibodies. Immunoglobulin (Ig) G fractions of three polyclonal rabbit antisera (Clinetics, Tustin, CA) were used with the following: 1) an antiwhole hCG-hLH (human luteinizing hormone) IgG, reacting with intact hCG-hLH and the free subunits; 2) an anti-hCGβ-hLHβ IgG, reacting with dimer and the free β-subunits; and 3) an anti-hCGα IgG, reacting with the dimer and the free α-subunit. Of 22 MoAb studied initially, 12 were selected for additional testing. These are listed in Table 1, which

**Table 1**

| **Nomenclature of Antibodies, Their Binding Sites, and Relative Specificities Toward Human Chorionic Gonadotropin, Its Subunits and Fragments** | | | | | | |
|---|---|---|---|---|---|---|
| Antibody | Immunogen | hCG holo | hCGα | hCGβ | hCGβ CTP | hCGβ frgmt |
| AS11 | hCG | + | - | + | + | NT |
| CTP101³⁹ | hCGβ(109 -145)-DT* | + | - | + | + | - |
| CTP102³⁹ | hCGβ(109 -145)-DT* | + | - | + | + | - |
| CTP103³⁹ | hCGβ(109 -145)-DT* | + | - | + | + | - |
| AS12 | hCG | + | - | + | - | NT |
| B108³⁸ | hCG† | + | - | + | - | + |
| B203²² | β-fragment | + | - | + | - | + |
| B107³⁸ | hCG | + | - | - | - | - |
| B109³⁸ | hCG | + | - | - | - | - |
| B201²² | β-fragment | - | - | + | - | + |
| B204²² | β-fragment | - | - | + | - | + |
| A105³⁸ | hCGα | + | + | - | - | - |
| +: Antibody binding to antigen with Ka, no less than 1 x 10⁷/mol; -: no measurable binding of antibody to antigen; NT: not tested; hCG: human chorionic gonadotropin; holo: intact; α: alpha subunit; β: beta subunit; CTP: carboxy terminal peptide. *Synthetic hCGβ-CTP, residues 109-145, conjugated with diphtheria toxoid. †Prefusion boost with hCGβ. All measurements were done by liquid-phase radioimmunoassays. ³⁹: Bibliographic reference | | | | | | |

also gives the nomenclature used for their identification, the immunogens used for their production, and their relative specificities. Details of the production and the characteristics of A105, B107, B108, B109, B201, B203 and B204 have been published (22, 38). The three CTP antibodies (CTP101, CTP102 and CTP103) are antibodies directed against epitopes of a synthetic hCGβ-CTP. They were obtained by using (as an immunogen) an anti-hCG vaccine consisting of a synthetic hCGβ-CTP, residues 109 to 145, conjugated to diphtheria toxoid (39). Details of the vaccine's preparation and the results of its effects and productions of anti-hCGβ-CTP antibodies in Phase 1 studies in humans, have been published (40-42). Secondary antibodies for all these reagents were goat anti-rabbit fluorescein isothiocyanate (FITC)-conjugated F(ab)₂ IgG (Tago, Burlingame, CA) for the polyclonal IgG, and goat anti-mouse FITC-conjugated F(ab)₂ IgG (Caltag, San Francisco, CA) for the MoAb.

### Preparation Of Cultured Cells For Flow Cytometry

1. Harvest the cells at confluence, using trypsin 0.25% with ethylenediamine tetracetic acid 0.1% when necessary, and collect them in a 15-ml or 50-ml tube.
2. Determine the number of cells per ml and the viability using a hemacytometer and trypan blue dye exclusion.
3. Pipette 10⁶ cells into a series of 12 x 75-mm polypropylene tubes, the number of tubes depending on the number of cells available and the number of antibodies to be used.
4. Pellet the cells by centrifuging at 200 x g for 7 minutes and discard the supernatant.
5. Add 100µl normal goat serum 3% in PBS to each tube and vortex gently for approximately 10-15 seconds. PBS was prepared from a stock solution of PBS 10X concentration (Whittaker, Walkersville, MD).
6. Incubate the tubes in ice for 30 minutes.
7. Suspend the cells in 3 to 3.5ml of PBS, vortex gently, and centrifuge suspension at 200 x g for 7 minutes. Discard the supernatant.
8. Resuspend the cells in PBS, vortex gently, and centrifuge them at 200 x g for 7 minutes. Discard the supernatant.
9. Add each primary antibody to its respective tube, following the plan of the experiment and using appropriate controls.
   a. The protein concentration (mg/ml) of each antibody is determined by the Bradford method (58) using the Bio-Rad™ protein assay according to the manufacturers instructions.
   b. The amount of antibody in micrograms of protein per 10⁶ cells must be determined experimentally by titration of the antibody with 10⁶ cells of the ATCC CCL 2 HeLa cell control.
10. Vortex the tubes gently and briefly.
11. Incubate overnight at 4 to 8°C on a dimensional rotator.
12. Wash the cells two times with PBS as in steps 7 and 8.
13. Add 100µl of appropriately diluted florescein isothiocyanate-conjugated species-specific second antibody. The concentration must be determined by titration.
14. Vortex the tubes gently and briefly.
15. Incubate in ice for 30 minutes.
16. Wash the cells twice with PBS as in steps 7 and 8.
17. Resuspend the cells in 0.5 ml paraformaldehyde 1% in PBS and vortex each tube gently. The cells can be stored at 4 to 8°C for one week at this point.
18. Use flow cytometry to determine the percent of cells binding with each antibody.

### Flow Cytometry

To determine the percentage of cells expressing hCG, its subunits and fragments, a Coulter Epics-C™ flow cytometer with a 2-W argon laser and standard settings and filters for FITC use was used. Instrument performance was controlled by daily calibration using the Coulter Immuno-Check™ system (fluorescence beads specially prepared and calibrated for fluorescence analysis). The percent of cells binding with each MoAb was determined using Coulter Immuno software that subtracts a control fluorescence profile from the test fluorescence profile when the two flow cytometry runs are superimposed.

To select the first antibodies to be used with this technique, the MoAb were tested for effects of antibody concentration on test sensitivity by titration and for effects of variability of epitope expression. The polyclonal IgG were tested only for effects of antibody concentration because, as a result of their polyclonality, they carry IgG recognizing different available determinants of hCG, thus making them more sensitive and relatively free of the effects of variability of epitope expression. The specificity of the polyclonal IgG was ascertained by using rabbit IgG as a control reagent for nonspecific binding and to obtain the control fluorescence profile. The control fluorescence profile for the MoAb was obtained by testing two unrelated MoAb of the same isotype for nonspecific binding, a mouse IgG1 Coulter Clone Control and an antileukocyte antigen (HLe-1) MoAb (Becton Dickinson, Mountain View, CA). These control reagents were used at the same concentration as the MoAb tested. The method was also tested for reproducibility at different levels of epitope expression and for effects of incubation time of the viability of the cells and on the percentage of cells reacting. Finally, to ascertain the specificity of the antigen-MoAb reaction, a competition Experiment was done by reacting 10⁶ HeLa cells with 150 µg of MoAb AS12 and different amounts of pure hCG. The ATCC CCL 2 HeLa cultured cells were used for all these tests with phosphate-buffered saline as the carrier for the cells and as the solvent for all antibodies.

### Discussion

It is stressed that there are fundamental differences between the flow cytometric method used here and all other techniques for detection and quantitation of hCG, its subunits and fragments. In the other types of analyses (liquid-phase or solid-phase radioimmunoassay and immunoradiometric assays), the antigen (hCG, its subunits or its fragments) is tested outside of its site of production (solubilized), and the results are expressed by the amount of antigen per unit of volume or weight. The flow cytometric technique used here uses live cells, and the results show the percent of cells analyzed which express the antigen on their cell membranes. The relative amount of antigen being expressed is given by its fluorescence intensity. These characteristics make the method presented here unique, and there is no comparison or relationship between the results that can be obtained here and those using any of the other analytical techniques.

The application of flow cytometry in concert with the use of well-defined standardized reagents results in a quantitative method for analyzing hCG, its subunits and fragments as membrane-associated material or, in other words, as a phenotypic characteristic of cultured human cancer cells. The method includes external and internal quality control, i.e., zero values obtained when the antigenic determinant is not available or is not being expressed in the cell surface, and the highest attainable degree of sensitivity. Because the technique is commercially available, it can be used anywhere. The specificity and quality assurance, which are superior to the best obtained by flow cytometric analysis of phenotypic markers (46), is caused in part by the fact that the antigen is one of the most widely studied glycoprotein hormones and is available with a high degree of purity (44).

### Conclusion

A quantitative flow cytometry method for the analysis of membrane-associated human chorionic gonadotropin (hCG), its subunits, and fragments on human cancer cells as used here uses a double-antibody reaction; a flow cytometer with a 2-W argon laser, standard settings, and filters for fluorescein isothiocyanate use; commercially available software; and the ectopic hCG producer CCL 2 HeLa cells from the American Type Culture Collection (ATCC) as a cell control to standardize the reagents and for overall quality control. Twenty-two monoclonal antibodies (MoAb) and immunoglobulin G fractions from three rabbit polyclonal antisera were tested for effects of antibody concentration (titration), reproducibility at different levels of epitope expression, and variability of epitope expression to select appropriate primary antibodies.

Based on the results of various tests, three polyclonal immunoglobulin G antibodies and a panel of nine MoAb directed to epitopes located in five different regions on the hCG molecule were selected as first antibodies. Their specificity was determined by using two unrelated MoAb of the same isotype at the same concentration to replace the primary MoAb and by a competition experiment. The unrelated MoAb were also used for the selection of the appropriate control fluorescence profile needed for the software. The unique characteristics of this method are the use of living cells, standardized reagents, internal and external quality control, and the high sensitivity, capable of allowing the detection of as few as 10³ molecules of fluorochrome per cell. Serial analyses of the ATCC CCL 2 HeLa cells and two of its variants of the eutopic hCG producer JEG-3 choriocarcinoma cells revealed the expression of membrane-associated epitopes of intact hCG, its subunits, and fragments by a high percentage of the cells, indicating that the expression of these sialoglycoproteins by these two different types of cancer cells is a common phenotypic characteristic.

### II. Expression Of Membrane-Associated Human Chorionic Gonadotropin, Its Subunits, And Fragments By Cultivated Human Cancer Cells

### Introduction

Because of the heterogeneity of hCG standard preparations and the great differences if immunoreactivity versus bioactivity reported in the literature (23), a reassessment of hCG, its biochemistry, and its association with malignant transformation and cancer was needed.

Reported here are the results of a systematic study of the expression of membrane-associated hCG, its subunits, and fragments by a wide variety of established, well-characterized cultured human malignant cell lines. Through this assessment, the following basic questions are addressed: 1) is the expression of membrane-associated hCG, its subunits, or fragments a phenotypic characteristic of some or all cultured cancer cells; 2) are there quantitative or qualitative differences in the expression of membrane-associated hCG-holo, its subunits, or fragments; 3) is the unique CTP of hCBβ always expressed in the cell membrane; and 4) do any of the MoAb used as reagents have any cytolytic or cytotoxic activity toward cancer cells in culture? To answer these questions, the flow cytometric procedure described supra was used which uses live cells with specific standardized reagents to achieve both the highest specificity and unsurpassed sensitivity characteristic of flow cytometry, with a level of detection of as little as 10³ molecules of fluorochrome per cell (30).

### Materials and Methods

### Cell Cultures

Seventy-four cell lines were studied. There were a total of 52 carcinomas, including 4 melanocarcinomas, 2 carcinomas of nasopharynx, 13 carcinomas of the lung (5 adenocarcinomas, 3 squamous cell, and 5 small cell), 6 carcinomas of breast, 7 carcinomas of urinary bladder, 8 squamous cell carcinomas of uterine cervix, 2 carcinomas of endometrium, 7 carcinomas of colon, and 3 carcinomas of prostate; 10 sarcomas (6 osteosarcomas and 4 rhabdomyosarcomas); 4 leukemias; 6 lymphomas; and 2 retinoblastomas.

All cells were grown in a humidified incubator at 37°C and 5% CO₂, in RPMI 1640 medium (JRH Biosciences, Lenexa, KS) with 10% defined iron-supplemented bovine calf serum (HyClone, Logan, UT), 2 mmol/1 L-glutamine (Fisher Pittsburgh, PA), 1 mmol/1 sodium pyruvate (JRH Biosciences), and 20 mg/1 of gentamicin sulfate (Sigma, St. Louis, MO). The 10% defined bovine calf serum was replaced with 1% Nutridoma™ (Boehringer Mannheim, Indianapolis, IN) in one experiment and with 10% decomplemented defined bovine calf serum (HyClone) in another. The cells were grown in 25-cm² tissue culture flasks and were detached using trypsin 0.25% with ethylene-diamine tetraacetic acid 0.1% (JRH Biosciences) or a cell scraper (Costar, Cambridge, MA) for passage or analysis. A hemacytometer was used to count the cells, and viability was determined by dye exclusion with trypan blue.

### Flow Cytometry

Expression of membrane-associated hCG, its subunits and fragments was determined using our flow cytometric technique. Briefly, the method involves a double-antibody reaction and the use of a Coulter Epics C™ flow cytometer (Hialeah, FL) with an argon laser and standard settings and filters for fluorescein isothiocyanate (FITC). Daily calibration for control of instrument performance was done using the Coulter Immuno-Check™ system. Coulter Immuno™ software, which subtracts a control fluorescence profile from the test fluorescence profile when the two flow cytometric runs are superimposed, was used to determine quantitatively the presence of cells binding with each antibody. ATCC CCL 2 HeLa cells were used as a positive cell control for hCG and its subunits. They were also used to standardize the polyclonal antibodies and MoAb. Polyclonal antisera, immunoglobulin (Ig) G fractions raised against the hCG dimer and both subunits, and a panel of MoAb recognizing different epitopes on the hCG-holo molecule, its subunits and fragments, were used as first antibodies, and goat anti-rabbit and goat anti-mouse FITC-conjugated F(ab)₂ IgG, respectively, as second antibodies. The purified IgG fractions from the polyclonal antisera, when used, gave a general assessment of the total expression of these particular membrane-associated glycoproteins. The MoAb were used to detect the expression of specific epitopes located at five different sites of the hCG molecule (22) on the membrane of the cultured cancer cells. These MoAb were directed against different conformational epitopes of hCG-holo (B109 or B107), hCGβ (AS12 or B108), hCGβ-free (B204 or B201), hCGβ-CTP (AS11 or CTP103), and an epitope of hCGα (A105). Unless indicated otherwise, polyclonal IgG against hCG-hLH was used at a concentration of 100 µg/10⁶ viable cells. All other polyclonal antibodies and MoAb were used at a concentration of 200 µg/10⁶ viable cells (except MoAb CTP103 which was used at a concentration of 300 µg/10⁶ cells). These saturating concentrations were defined by titrations of the antibodies using CCL 2 HeLa cells.

To study the effects of a relatively low viability (under the same conditions of culture) on the flow cytometric analysis, cells from a retinoblastoma cell culture were analyzed simultaneously with and without propidium iodide (PI) treatment (10⁶ cells treated with 10 µl of 0.5 mg/ml PI in phosphate-buffered saline for 5 minutes). Although PI and FITC are excited by the argon laser at the same wavelength (488 nm), their emission wavelengths are different, i.e., 520 nm (green) for FITC and 615 nm (orange-red) for PI. This permits exclusion of the dead PI-containing cells (30).

### Results and Discussion

Results obtained from analyses of the 74 cancer cell lines are given in Tables 2 to 16. All cell cultures were grown in the same media under the same parameters to eliminate or minimize effects of the conditions of culture, an important factor which must be considered (45). The viabilities obtained in each passage of each cell line, using the same culture conditions, were notable considering the great diversity in type and origin of the cultured cells. Thus, viability ranged from 90% to 100% in 137 passages from 67 of the cell lines and from 70% to 89% in 18 passages of 13 cell lines. Five passages of four of these cultures had viabilities of 70% to 79%, permitting study of the effects of a relatively low viability on the results of the flow cytometric analyses. It was not possible to do this during the development of the method because of the consistently high viability of the HeLa cells used to standardize the reagents and technique.

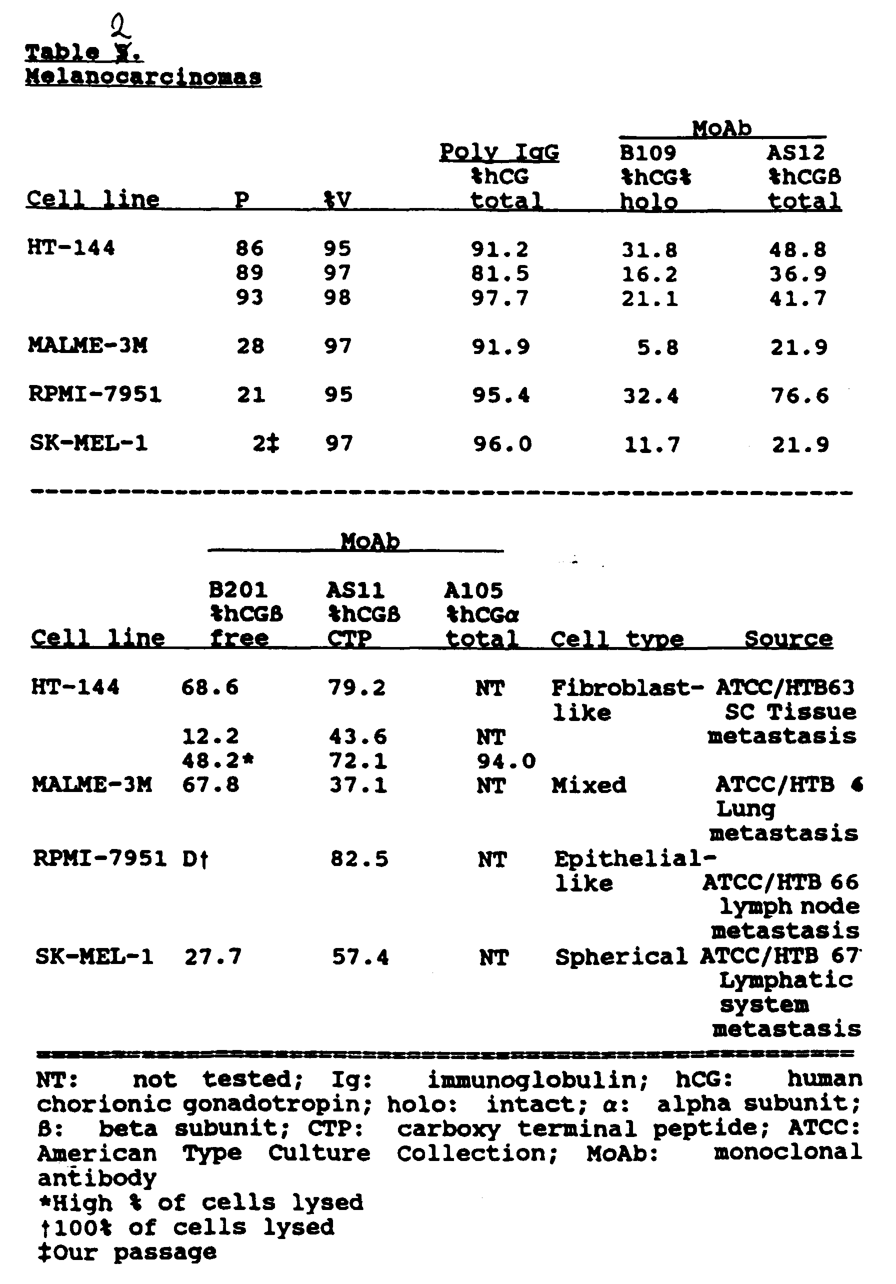

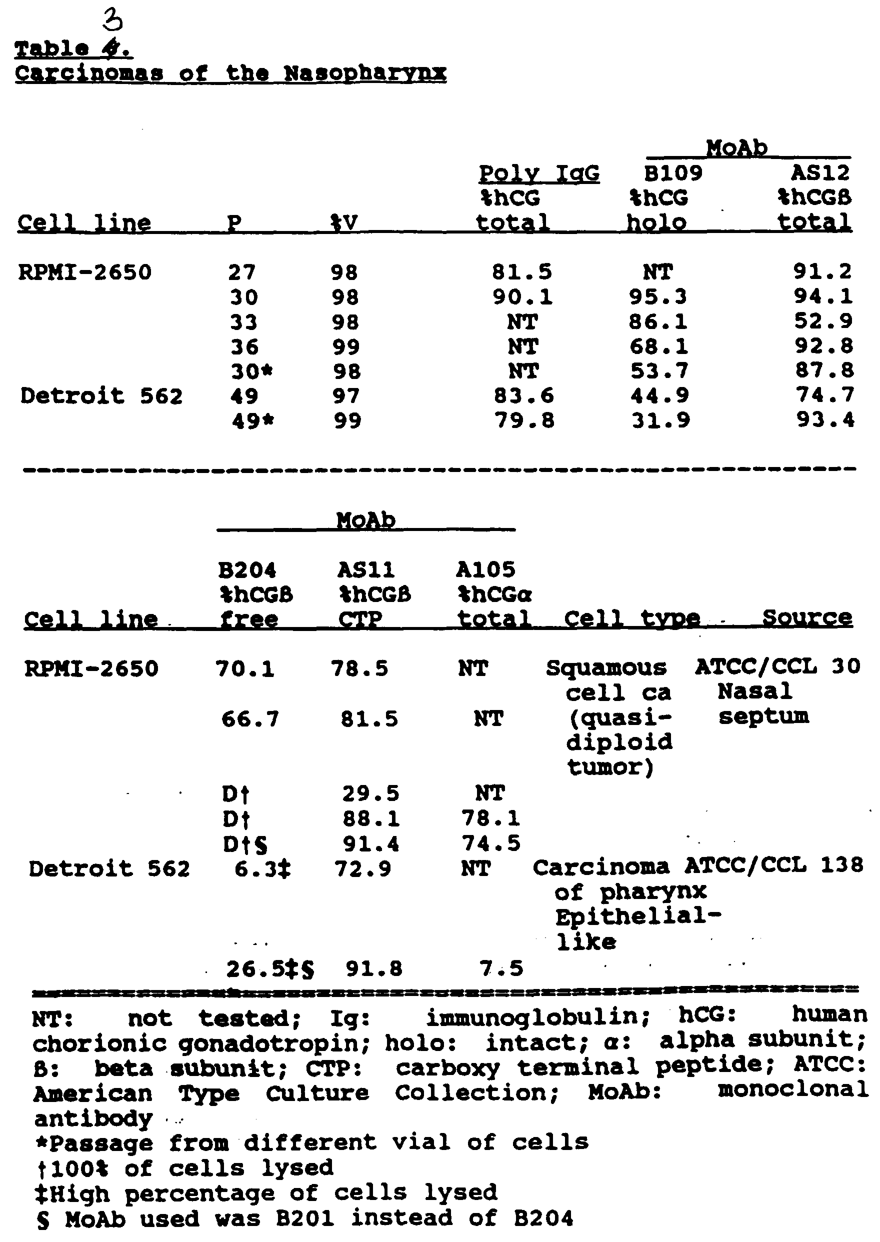

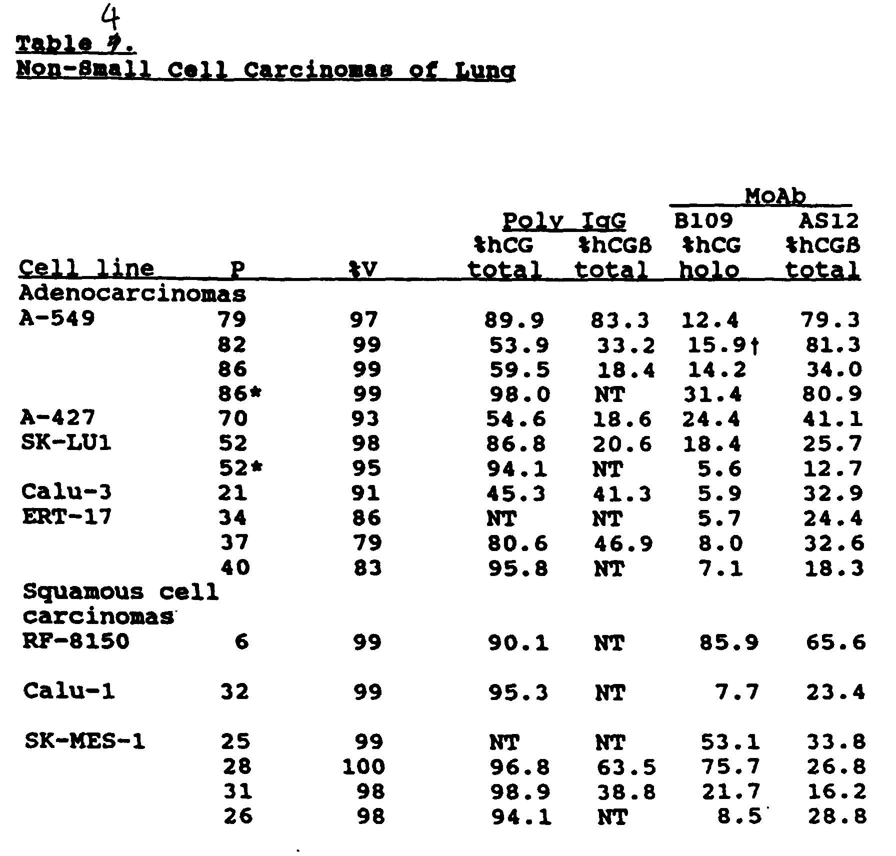

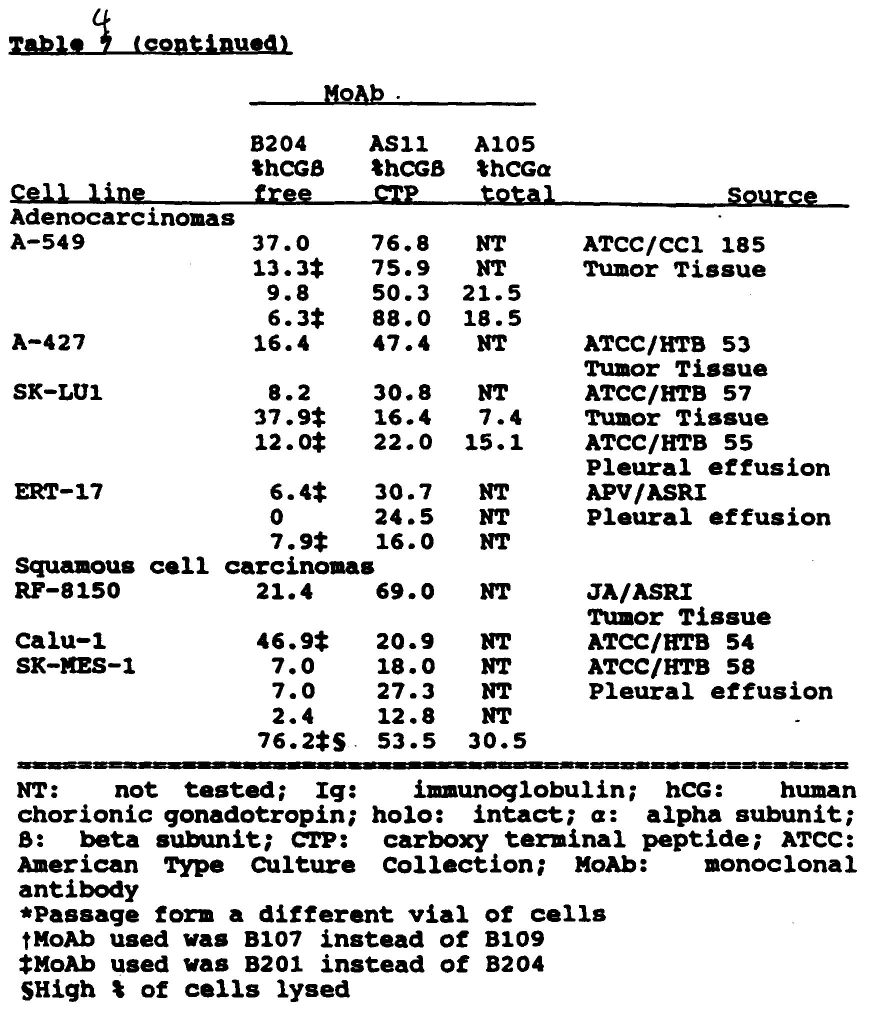

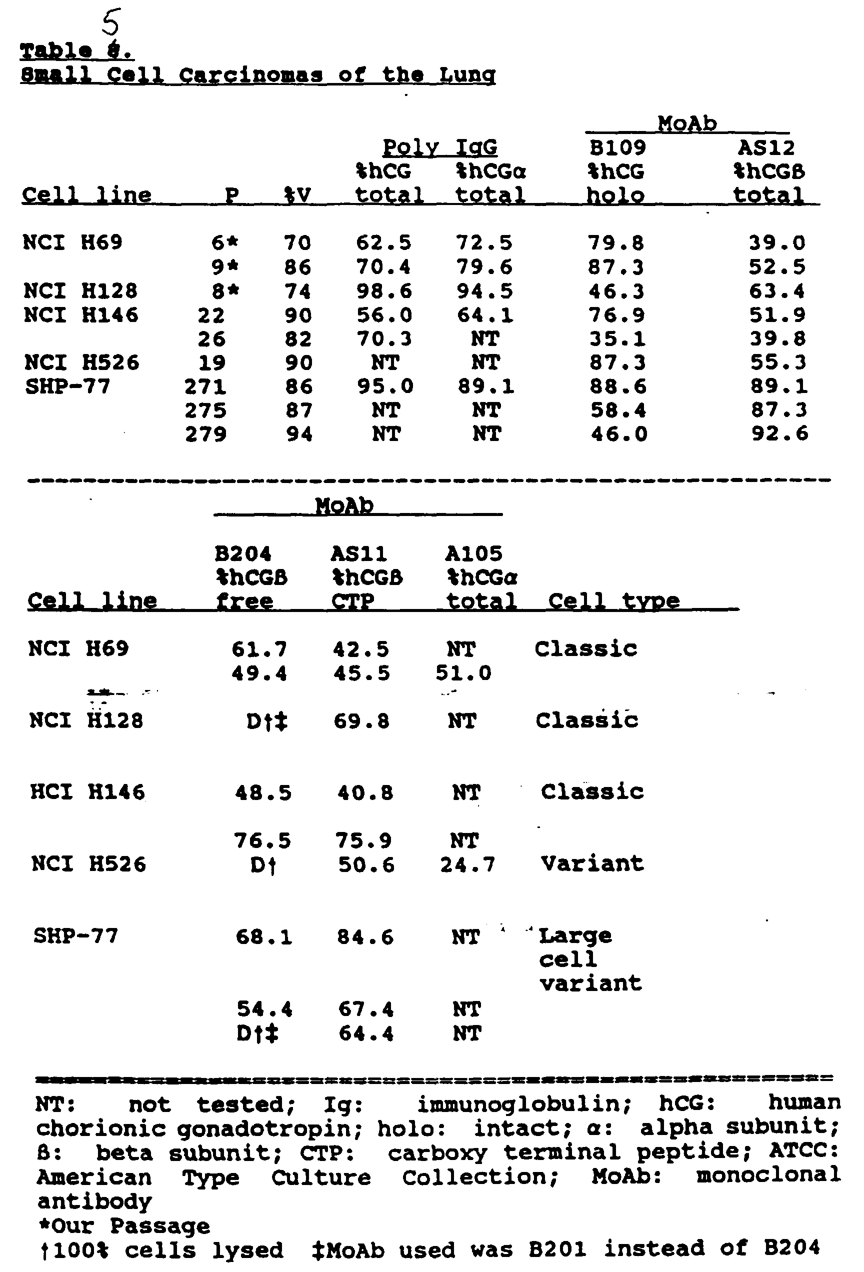

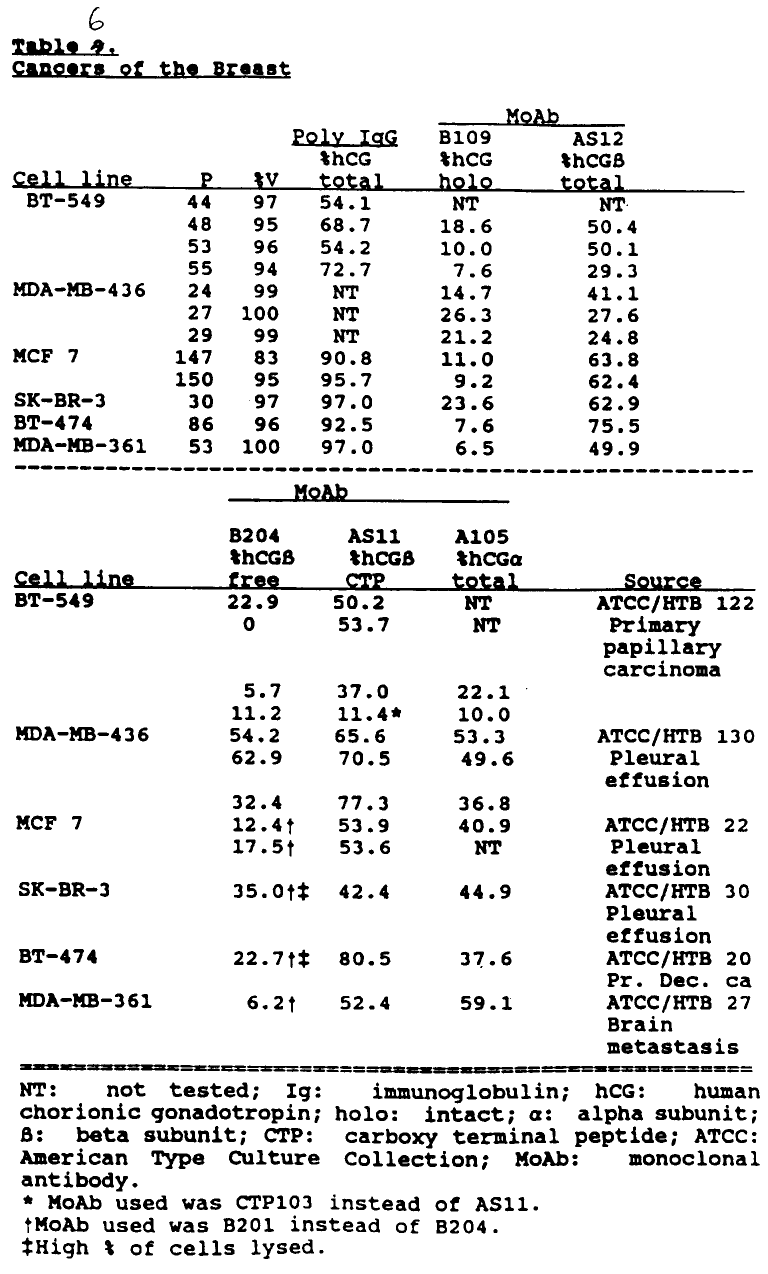

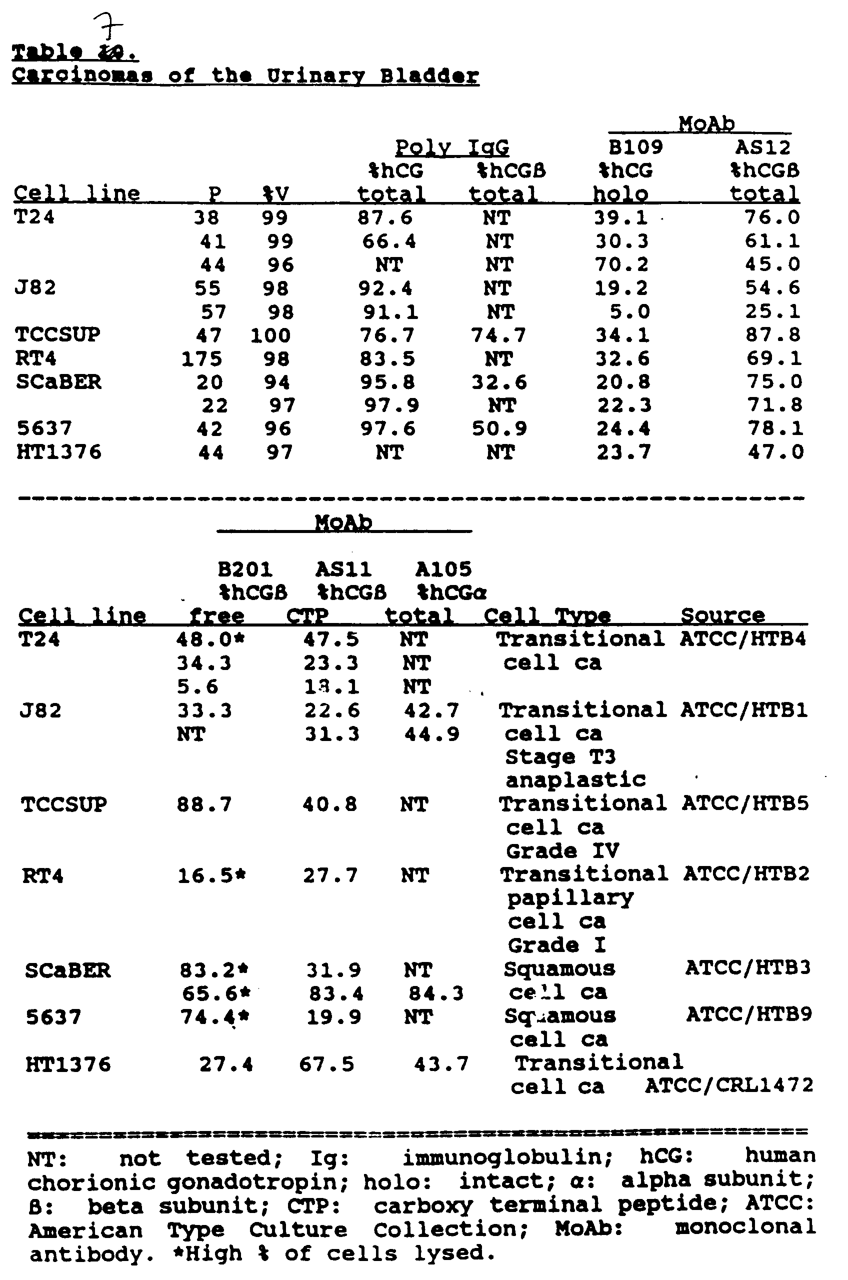

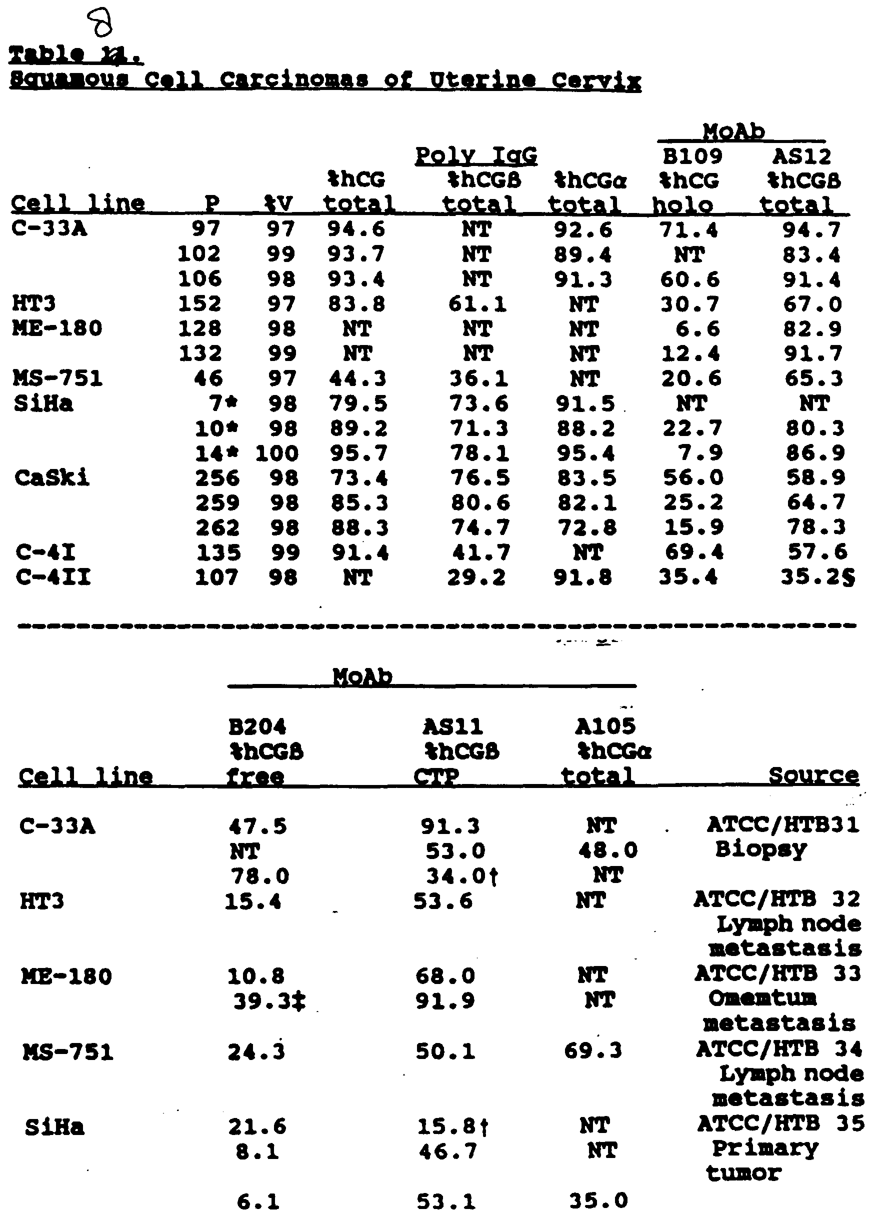

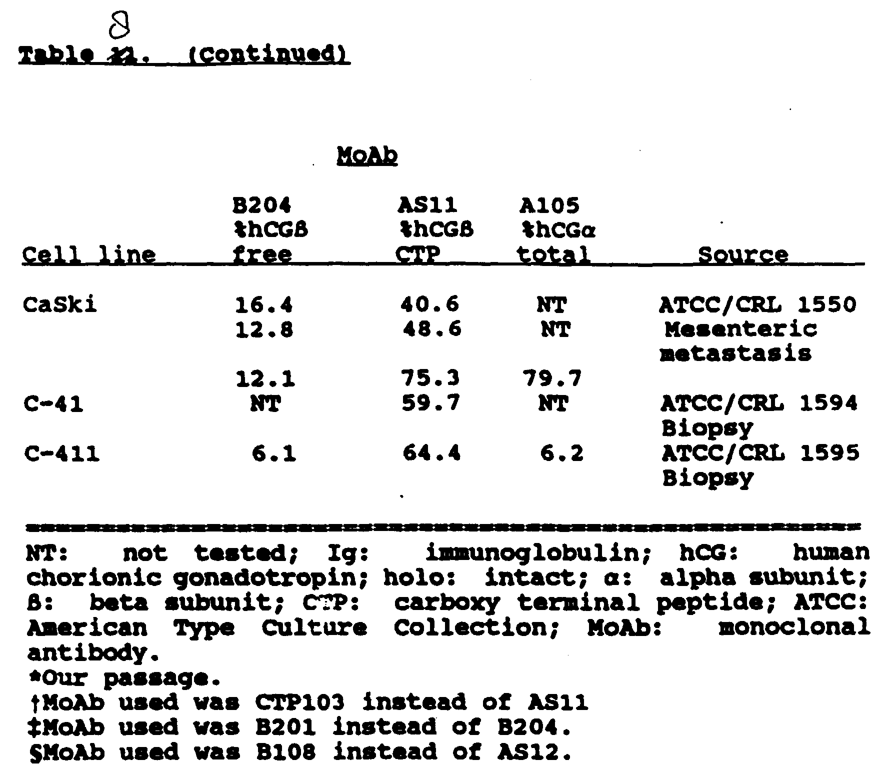

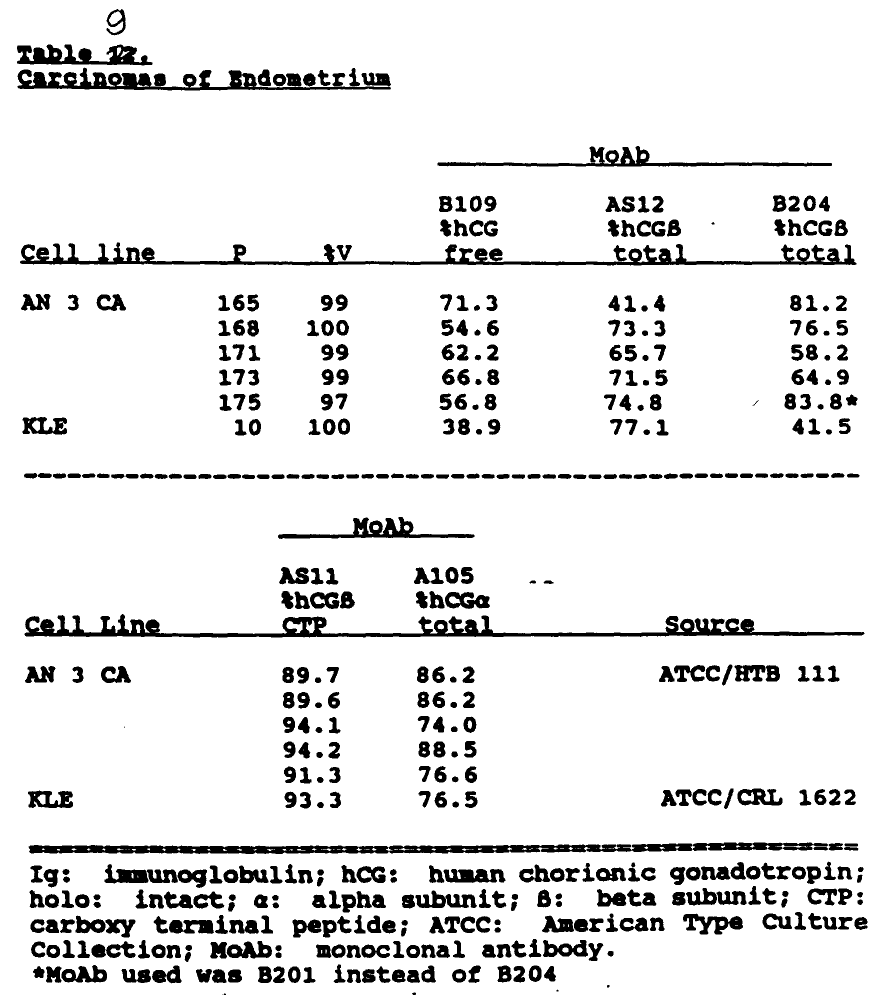

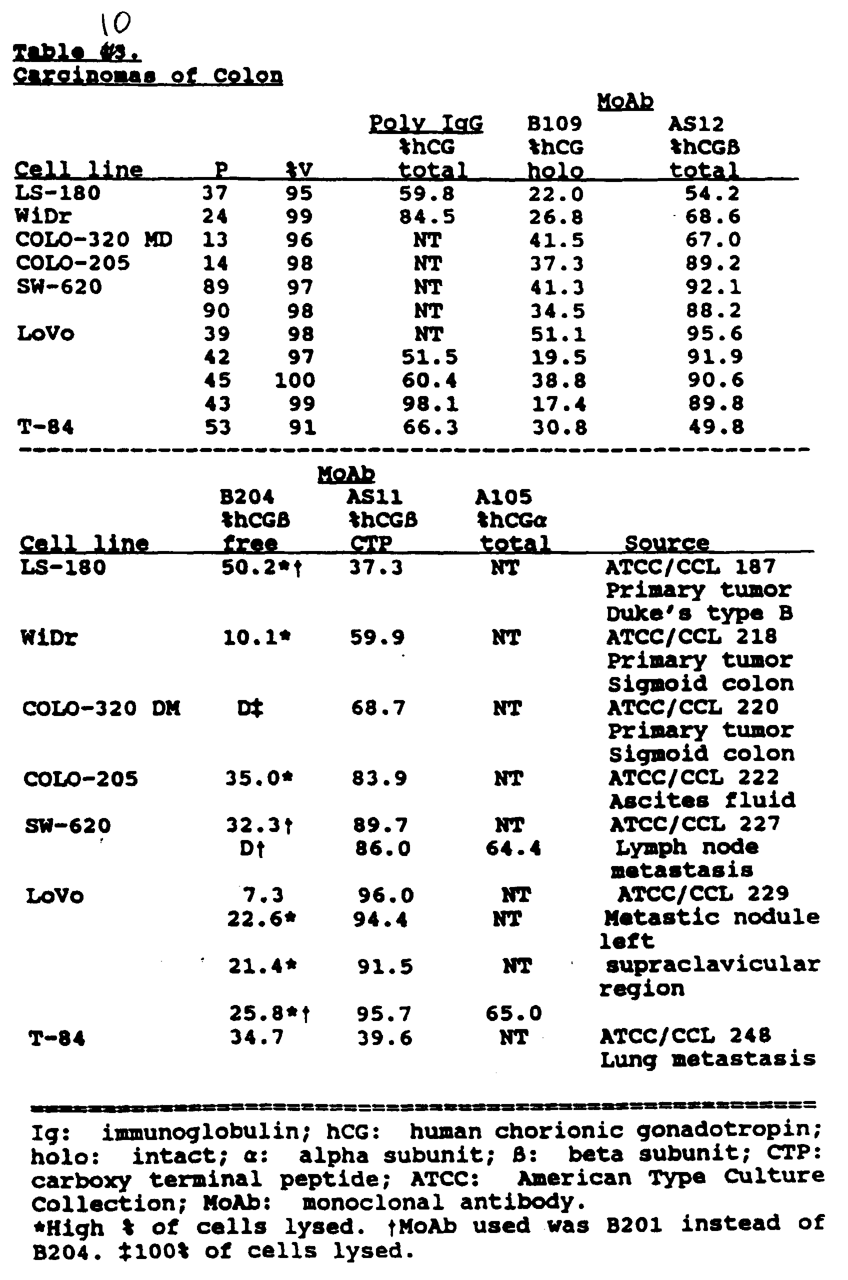

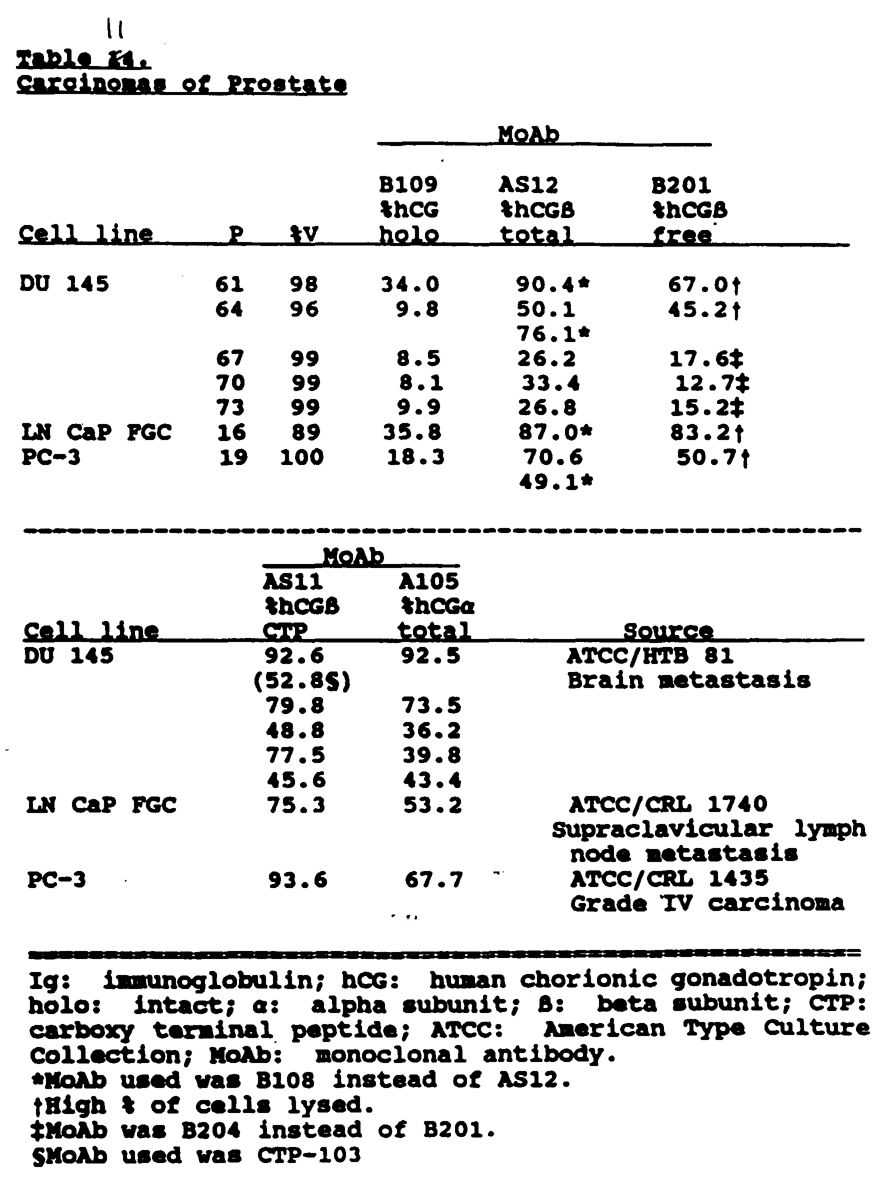

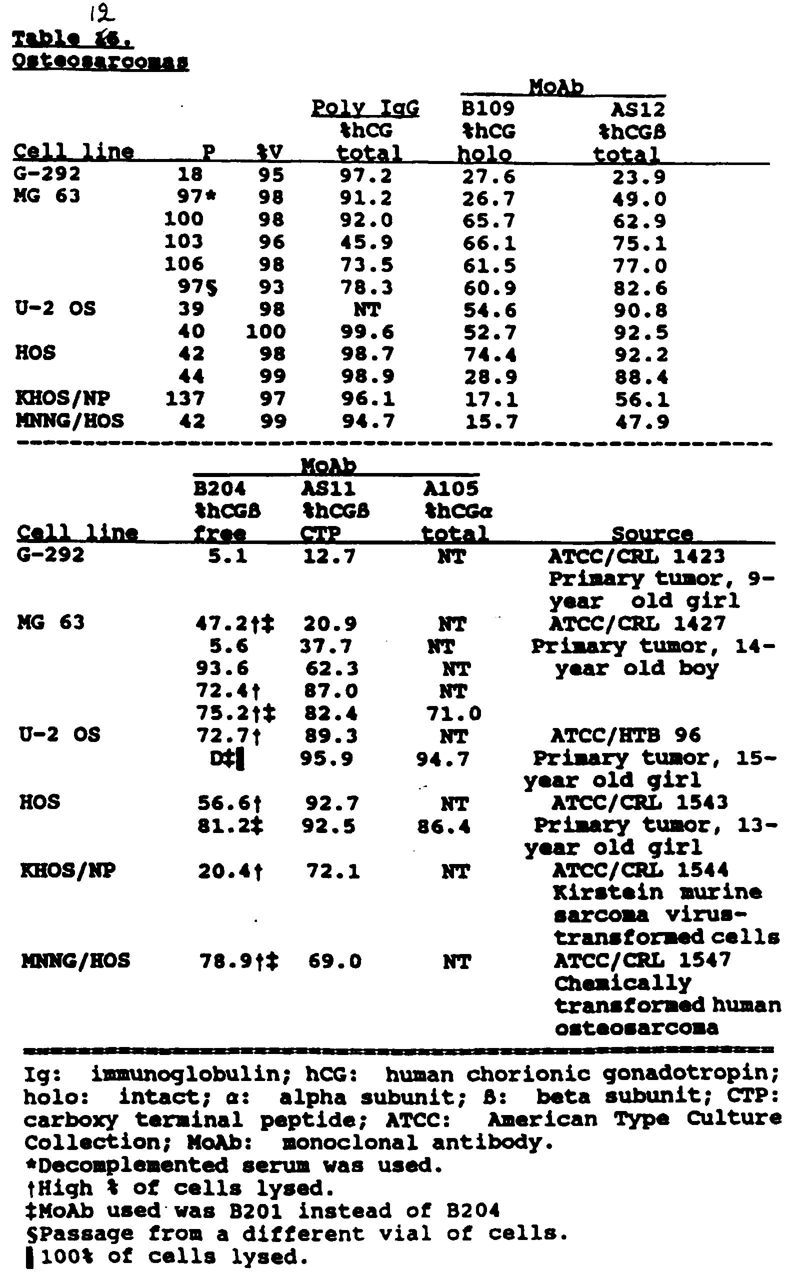

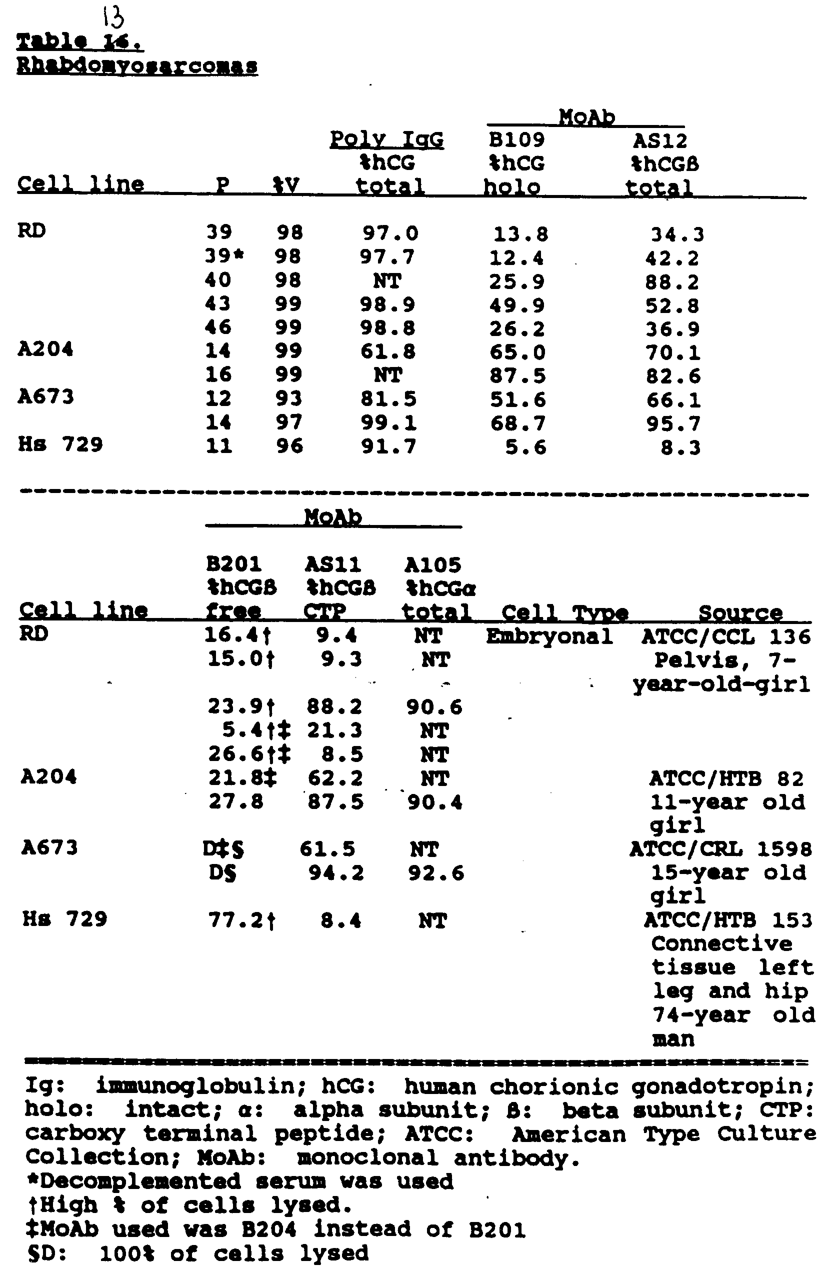

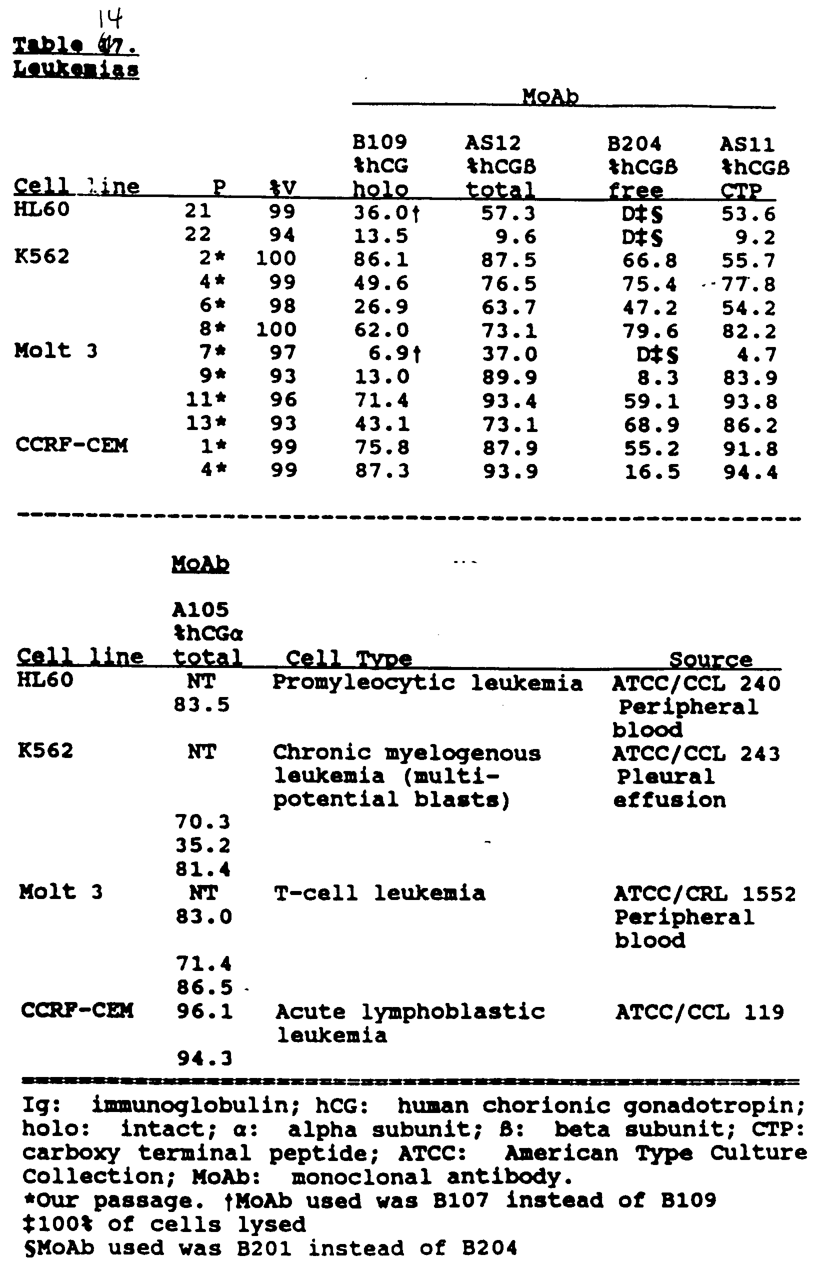

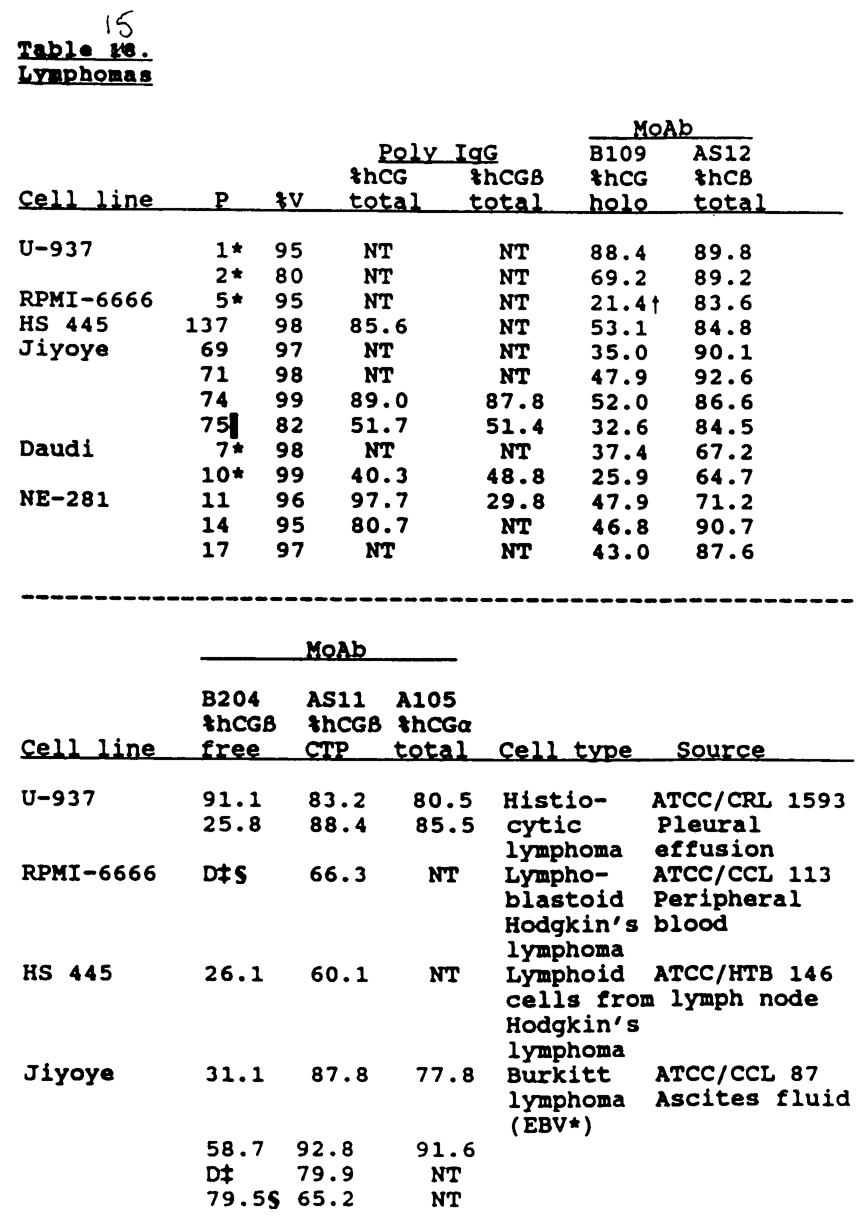

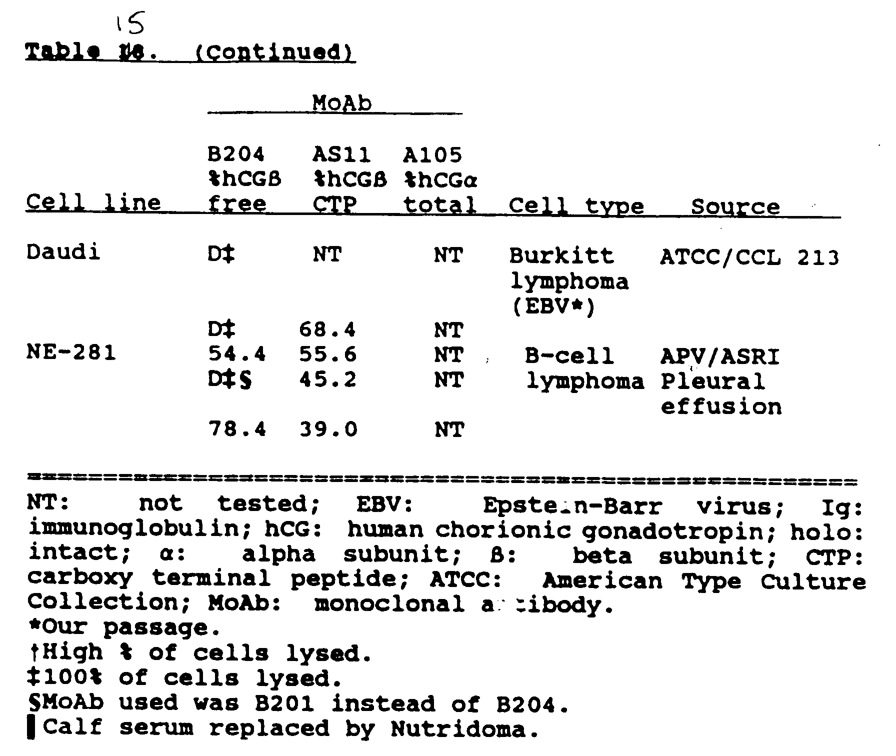

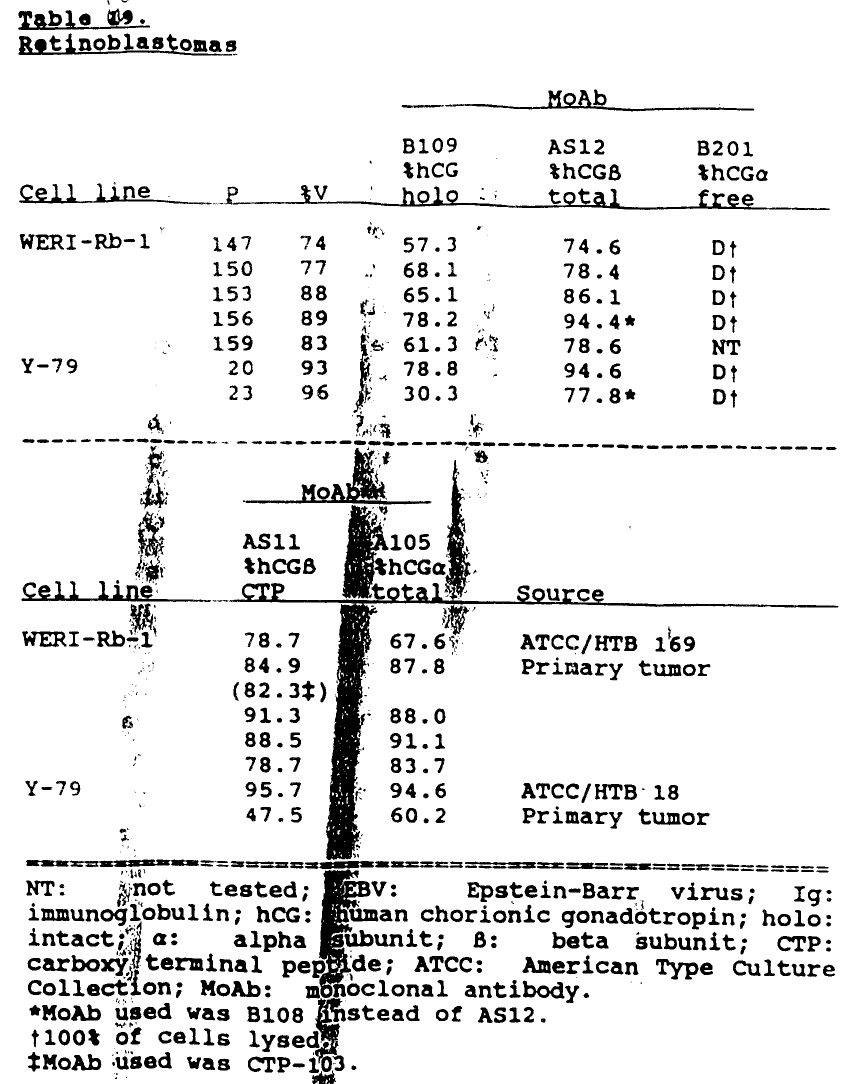

The results of serial analyses done in all cancer cell lines (Tables 2 to 16) showed expression of epitopes of hCG-holo, its subunits, and fragments by cells from all cell fines studied. Variable degrees of reactivity were seen. These variations in the expression of some of the membrane-associated hCG epitopes ranged from more than 90% of the cells to 0%, as in the case of one of the determinants of the free hCGβ recognized by MoAb B204 and illustrated by Passage 1 of the histiocytic lymphoma cell line U-937, ATCC CRL 1593 (Table 15). Here, reactivity was found in 91.1% of cells, while 0% values were shown by Passage 37, one of three passages studied of the adenocarcinoma of lung cell line ERT-17 (Table 4), and by Passage 48, one of four passages studied of the adenocarcinoma of breast cell line BT-549, ATCC HTB 122 (Table 6). All these values, obtained with cells grown under the established standard conditions of culture, indicate that expression of the membrane-associated epitopes of hCG, its subunits and fragments was a phenotypic characteristic of the cell and not a product of growth conditions. Moreover, although in this technique a value of less than 10% with low fluorescence intensity was considered to be 0%, an instrument value of 0% for the expression of a particular epitope in one of several passages of the same cell line gave additional proof of the specificity of the reagents considering that all MoAb used (except B201) are of the same isotype, IgG1. The MoAb B201 has an IgG2 isotype.

The conformational epitope of the intact hCG recognized by MoAb B109 was expressed by a variable percent of cells from all cell lines in the 151 passages in which this determinant was investigated. Because this epitope is expressed only in hCG-holo, these results give strong evidence for the synthesis of both competent subunits (17-19) by all human cancer cells in culture. There was also a striking quantitative difference in the expression of hCG-holo by different types of cancers. In 34 passages of 18 lines of melanocarcinomas, adenocarcinomas of lung, adenocarcinomas of breast, and carcinomas of prostate (Tables 2, 4, 6 and 11, respectively), identified as Group 1 hereafter, the means of the percent of cells of each type of cancer expressing the determinant were 19.83% ± 4.39 (mean ± standard error of the mean; range, 5.8% to 32.4%), 13.54% ± 2.56 (range, 5.6% to 31.4%), 14.87% ± 2.25 (range, 6.5% to 26.3%), and 17.77% ± 4.61 (range, 8.5% to 35.8%), respectively. None of these means were statistically different from one another based on analysis of variance followed by Scheffe's test for pairwise comparisons (56). P values of 0.05 or less were considered significant.

A second group consisting of 11 passages of seven carcinomas of colon cell lines (Table 10) and 11 passages of seven carcinomas of urinary bladder cell lines (Table 7) showed a mean expression of 32.81% ± 3.18 and 29.24% ± 4.93 within a relatively narrow range of expression of this determinant, 17.4% to 51.1% and 5.0% to 39.1%, respectively, with only one value, 70.2% (Table 7), failing outside this range. As in Group 1, their mean values were not significantly different.

By contrast, in 28 passages of 11 cultures of carcinomas of nasopharynx (Table 3), small cell carcinomas of lung (Table 5), carcinomas of endometrium (Table 9), and retinoblastomas (Table 16), identified as Group 3 hereafter, the mean values of the percent of cells of each type of cancer expressing the conformational determinant were 63.33% ± 9.97 (range, 31.9% to 95.3%), 67.3 ± 6.98 (range, 35.1% to 88.6%), 58.4% ± 4.64 (range, 38.9% to 71.3%), and 62.72% ± 6.20 (range, 30.3% to 78.8%) respectively, demonstrating the highest degree of expression of hCG-holo. As in groups 1 and 2, none of the means in Group 3 were significantly different from each other.

Analysis of variance and Scheffe's test for pairwise comparison done on the means of the mean values of the three groups, 15.91% ± 1.59 (Group 1), 31.02% ± 2.89 (Group 2), and 63.41% ± 3.46 (Group 3), demonstrated that the differences among these means were statistically significant. In the other 31 cell lines studied, corresponding to six types of cancer (Tables 4, 8 and 12 to 15), the variability of expression of the conformational epitope in the different passages of each of the cell lines of each cancer was too great to permit any grouping and comparison (e.g., range, 5.6% to 87.5%).

The expression of any single epitope on the surface of a cell depends on several factors. Because conditions of growth were eliminated or minimized as a variable, the only other factors to be considered in our studies (in addition to the inherent phenotypic variabilities from the kinetics of cell membrane synthesis) were the different kinetics of hCGα and hCGβ synthesis. In the particular case of the specific conformational epitope of hCG-holo, the need for the biosynthesis of the two competent subunits was also a factor (16-19). The different kinetics of the transcription of the genes encoding hCGα and hCGβ (14, 15) were not sufficient to explain the low expression of the conformational determinant of hCG-holo in the cancers in Group 1 (melanocarcinoma (Table 2), carcinoma of breast (Table 6), and carcinoma of prostate (Table 11)) because epitopes of hCGα and hCGβ-CTP were expressed by a relatively high percentage of cells in all corresponding passages. This was demonstrated by the reactivity of these cells with MoAb A105 and AS11, respectively. The only exception in this group was the carcinoma of lung cell lines (Table 4), which also had a low expression of the epitope of hCGα (A105). These results showed that it was logical to study the distribution of the expression of the specific epitopes of hCGβ and hCGα.

In Group 1, the difference of the means of the expression of each determinant was significantly different for each type of cancer, indicating that the cells of the cancers in this group, with the exception of the adenocarcinomas of lung, produced a significant amount of modified (incompetent) hCGα and hCGβ because of the elevated values of hCGβ and hCGα. By contrast, in the adenocarcinomas of lung cells, the limiting factor for the formation of hCG-holo appeared to be the low synthesis of hCGα. The values for Passage 55 of the carcinoma of breast line BT-549 (Table 6) were excluded from statistical calculation because the MoAb CTP103 (against the synthetic hCGβ-CtP) was used instead of MoAb AS11. Carcinomas of colon in Group 2 and carcinomas of endometrium and retinoblastomas in Group 3, as in these carcinomas of Group 1, also appeared to produce considerable quantities of incompetent subunits. By contrast, the cells from carcinomas of nasopharynx and small cell carcinoma of lung in Group 3 and carcinoma of urinary bladder in Group 2 appeared to produce a significantly lower amount of incompetent subunits.

The expression of the epitope of the unique hCGβ-CTP, recognized by MoAb AS11 by all cell lines in all passages investigated, was higher than the expression of hCGα in most cases, providing additional evidence that the material being recognized in the cell membrane of the cultured cancer cells was related to hCBβ, free or as part of hCG-holo. Additional evidence was also provided by the expression of the epitope recognized by MoAb CTP103 in the four instances in which this MoAb against a synthetic hCGβ-CTP (residues 109 to 145) was used: a carcinoma of breast (BT-549, ATCC HTB 122, Table 6), two squamous cell carcinomas of uterine cervix (C33A, ATCC HTB 31, and SiHa, ATCC HTB 35, Table 11), and a prostatic carcinoma (DU-145, ATCC HTB 81, Table 14).

Others (37) recently reported that they were unable to detect hCGα mRNA in CaSki cells (ATCC CRL 1550, Table 11), a cell line obtained from mesenteric metastases of a patient with a squamous cell carcinoma of the uterine cervix, in which synthesis and secretion of hCGβ and bioactive hCG was reported several years ago. The results presented here demonstrate expression of membrane-associated conformational epitopes of hCG-holo (range, 15.9% to 56.0% of the cells in three passages) and expression of the epitope of hCGα recognized by MoAb A105 (by 79.7% of the cells), with a high expression (range, 40.6% to 75.3%) of hCGβ-CTP determinants. These authors (37) admitted that some of their results may have only reflected conditions of cell culture or, more probably, the lack of sensitivity of the methology because no poly-A mRNA was analyzed nor was the polymerase chain reaction technique.

An unexpected finding was the observation that MoAb directed to epitopes of hCGβ-free (B201 or B204) are able to lyse cells. These effects were remarkable in the sarcomas (Tables 11 and 12), lymphomas (Table 14), and retinoblastomas (Table 15). In several instances, there were no cells left to be analyzed by flow cytometry (indicated by "D" in the tables). These effects were also observed in some passages of carcinoma cell lines, melanocarcinomas, carcinomas of nasopharynx, small cell carcinomas of lung, and carcinomas of urinary bladder, colon, and prostate (Tables 2, 3, 5, 7, 10, and 12, respectively).

The RD rhabdomyosarcoma cell line (ATCC 136, Table 13) grown in medium with defined bovine calf serum with and without complement and analyzed concomitantly in a duplicate experiment, and in another experiment, the Jiyoye Burkitt lymphoma cell line (ATCC CCL 87, Table 15) grown in serumless medium (Nutridoma™, showed that the cytolytic activity appeared to be independent of complement.

The technique and results presented here cannot be compared with those of others also working with human cell lines, not only because of the different in specificity and sensitivity of the flow cytometric method, but also because their analyses were done on extracts of media (58, 59), reflecting only the secretory pool, or on extracts on media and cell lysates (60, 61), reflecting the secretory pool and the total cell pool (i.e., cytosol and membrane pools alone or in combination with the secretory pool). In this respect, it was stated that these types of glycoproteins can be found in the cytosol extracts, although they are not detectable in the media, a finding which may invalidate the negative results obtained by analyzing culture media alone (or blood serum) and the conclusions based on such results (61).

In summary, our studies thus far have demonstrated several things. First, determinants of membrane-associated hCG-holo, its subunits and fragments in cultured human cancer cells are not a product of growth conditions but are a phenotypic characteristic of all cell lines investigated, irrespective of type or origin. Second, the cultured cells from a defined group of carcinomas and the retinoblastomas showed quantitative and qualitative differences in the expression of the membrane-associated hCG-holo, its subunits and fragments. Thus, melanocarcinomas, adenocarcinomas of lung, and carcinomas of breast and prostate cell lines (Group 1) appear to express a relatively low amount of membrane-associated hCG-holo. Concomitantly, but with the exception of the adenocarcinoma of lung cells, large amounts of modified free hCGα and/or hCGβ are expressed, as indicated by the high percentage of cells reacting with subunit-recognizing MoAb. Adenocarcinomas of colon cell lines in Group 2 and retinoblastomas and carcinomas of endometrium in Group 3 appeared to follow the same trend of expression, by contrast with the other cell lines in Group 2 and retinoblastomas and carcinomas of endometrium in Group 3 appeared to follow the same trend of expression, by contrast with the other cell lines studied. Adenocarcinoma (non-small cell carcinoma) of lung appeared to be the only type of cultured cancer cells in which the limiting factor for the expression of intact hCG was the low degree of hCGα synthesis. Third, the unique CTP was always expressed in the membrane of a high percentage of cells of all cancer cell lines studied, indicating that the membrane-associated material being recognized by both MoAb AS11 and CTP103, was hCGβ, free or as part of hCG-holo. Fourth, the experiments in which MoAb directed against hCGβ-free determinants were used, revealed an in vitro complement-independent cytolytic activity for some types of the cultured cancer cells used.

### Conclusion

The expression of human chorionic gonadotropin (hCG), its subunits, and fragments on the cell membrane of cultured human cancer cells was investigated using a flow cytometric method. This method uses living cells; a double-antibody reaction; a flow cytometer with an argon laser, standard settings, and filters for fluorescein isothiocyanate; commercially available software; the American Type Culture Collection (ATCC) CCL 2 HeLa cell line as a cell control and overall quality control; polyclonal rabbit antisera raised against the hCG dimer, its α subunit (hCGα), and its β subunit (hCGβ); and a panel of monoclonal antibodies (MoAb) recognizing different epitopes on the intact hCG molecule, its subunits, and fragments. The purified immunoglobulin G fractions from the polyclonal antisera were used to estimate the total expression of the membrane-associated glycoproteins; the MoAb were used to detect the expression of epitopes of the hCG dimer, it subunits and fragments. The results of the analyses done on cells from 74 established cancer cell lines of different types and origins (including 52 carcinomas, 10 sarcomas, 4 leukemias, 6 lymphomas, and 2 retinoblastomas) showed variable degrees of reactivity in a great percentage of cells in all cell lines studied with MoAb directed against different conformational epitopes of intact hCG (hCG-holo), hCGβ, hCGβ-free, the carboxy terminal peptide (CTP) of hCGβ, and an epitope of hCGα. The expression of the membrane-associated epitopes of hCG and its subunits was found to be a phenotypic marker characteristic of all evaluated cultured human cancer cell lines, irrespective of their type or origin. There were, however, quantitative and qualitative differences in the expression of the different epitopes. Thus, hCGβ, free and as part of hCG-holo, recognized by the MoAb against hCGβ-CTP, was expressed by a high percentage of cells in most lines. There was a great variability in the expression of hCG-holo, recognized by the MoAb B109. For this reason some groups of cancers expressed larger amounts of incompetent hCGα and/or hCGβ than others. Cell lines derived from adenocarcinomas of the lung were the only exception to this general finding. The expression of small amounts of hCG-holo was caused by a low degree of hCGα synthesis.

### III. In Vivo Expression Of Membrane-Associated Human Chorionic Gonadotropin By Cells Isolated From Cancerous Human Tissues

The expression of hCG, its subunits and fragments by live cancer cells isolated from 15 malignant human neoplasms (6 lung neoplasms, 3 breast neoplasms, 3 colon neoplasms, one kidney neoplasm, one urinary bladder neoplasm, and one testicular neoplasm) was demonstrated using a flow cytometry method and a panel of monoclonal antibodies specific for epitopes from 5 different sites on the hCG molecule (59). In contrast to the cells isolated from the malignant neoplasms, cells isolated from a benign neoplasm (uterine leiomyoma) showed no measurable hCG-related determinants. These results, similar to those seen with cultured human cancer cells (60), indicate that the in vivo expression of hCG, its subunits and fragments is a common phenotypic characteristic of cancer cells of different types and origins.

### References

1. Fiddes, J.C., Goodman HM. Isolation cloning and sequence analysis of the cDNA for the α-subunit of human chorionic gonadontropin. Nature 1979; 281:351-356.
2. Boothby M, Ruddon RW, Anderson C, McWilliams D. Boime I. A single gonadotropin α-subunit gene in normal tissue and tumor-derived cell lines. J. Biol. Chem. 1981; 256:5121-5127.
3. Naylor S.L., Chin W.W., Goodman H.M., Lalley, PA. Grzechik K.H. Sakaguchi AY. Chromosomal assignment of genes encoding the α and β-subunits of gonadotropin hormones in man and mouse. Somat Cell Mol Genet 1983; 9:757-770.
4. Fiddes J.C., Goodman H.M. The c-DNA for the β-subunit of human chorionic gonadotropin suggests evolution of gene by readthrough into the 3' untranslated region. Nature 1980; 286:684-687.
5. Policastro P. Daniels-McQueen S, Carle G, Goime I. A map of the hCGβ-LHβ gene cluster. J. Biol. Chem. 1986; 261:5907-5916.
6. Pierce J.G. Parsons TF. Glycoprotein hormones: Structure and function. Annu Rev Biochem 1981; 50:465-495.
7. Acevedo, H.F., Slifkin M, Pouchet GR, Rakhshan M. Idnetification of the β-subunit of choriogonadotropin (CG) in human spermatozoa. In: Troen P, Nankin H.R, eds. The Testes in Normal and Infertile Men. New York: Raven, 1977:1985-1992.
8. Ash R.H, Fernandez E.D, Siler-Khodr TM, Pauerstein CJ. Presence of hCG-like substance in human sperm. Am J. Obstet Gynecol 1979; 135:1041-1047.
9. McGregor W.G., Kuhn R.W., Jaffe R.B. Biologically active chorionic gonadotropin: Synthesis by the human fetus. Science 1983; 220:306-308.
10. Saxena R.R., Haour F., Schmidt-Gollwitzer M. Radioreceptor assay of human chorionic gonadotropin: Detection of early pregnancy. Science 1974; 194:793-795.
11. Tesarik J. Kopecny V., Planchot M., Mendelbaum J. Activation of nucleolar and extranucleolar RNA synthesis and changes in the ribosomal content of human embryos developing in vitro. J. Reprod Fertil 1986; 78:463-470.
12. Braude P.R. Gene activation in early human development. Hum Reprod 1987; (Suppl 1) 2:29.
13. Bonduelle M.L., Dodd R., Liebaers I., Van Steuteghen A., Williamson R., Akhurst R. Chorionic gonadotropin-β mRNA, a tropoblast marker, is expressed in human 8-cell embryos derived from triponucleate zygotes. Hum Reprod 1988; 3:909-914.
14. Cole L.A., Hussa R.D., Rao CVO. Discordant synthesis and secretion of human chorionic gonadotropin and subunits by cervical carcinoma cells. Cancer Res 1981, 41:1615-1619.
15. Milsted A., Cox R.P., Nelson J.H. Cyclic AMP regulates transcription of the gene encoding human chorionic gonadotropin with different kinetics. DNA 1987; 6:213-219.
16. Cole L.A., Perini F., Birken S., Ruddon R.W. An oligosaccharide of the O-linked type distinguishes the free from the combined form of hCGα subunit. Biochem Biophys Res Commun 1984; 122:1260-1267.
17. Nishimura R., Shin J., Ji I et al. A single amino acid substitution in an ectopic α-subunit of a human carcinoma choriogonadotropin. J. Biol Chem 1986; 261:10475-10477.
18. Cox G.S., Rimerman R.A. Purification and characterization of the gylcoprotein hormone α-subunit-like material secreted by HeLa cells. Biochemistry 1988; 27:6474-6487.
19. Beebe J.S., Mountjoy K. Krzesicki R.F. Perini F. Ruddon R.W. Role of disulfide bond formation in the folding of human chorionic gonadotropin β-subunit into an α β dimer assembly-competent form. J. Biol Chem 1990; 265:312-317.
20. Locke Y.W. Immunology and Immunopathology of the Human Fetal-Maternal Interaction: Special surface properties of human trophoblast cells. New York:Elsevier-North Holland, 1978; 16-26.
21. Birken S., Armstrong E.G., Kolks MAG at al. Structure of the human chorionic gonadotropin β-subunit fragment from pregnancy urine. Endocrinology 1988; 123:572-583.
22. Krichevsky A., Armstrong E.G., Schlatterer J., et al. Preparation and characterization of antibodies to the urinary fragment of the human chorionic gonadotropin β-subunit. Endocrinology 1988: 123:584-593.
23. Wehmann R.E., Blithe D.L., Akar A.H., Nisula BC. β-Core fragments are contaminants of the World Health Organization reference preparations of human choriogonadotropin and its α-subunit. J. Endocrinal 1988; 117:147-152.
24. Pollak S., Halpine S., Chait B.T., Birken S. High resolution high performance liquid chromatography fingerprinting of purified human chorionic gonadotropin demonstrates that oxidation is a cause of hormone heterogeneity. Endocrinology 1990; 126: 199-208.
25. Kardana A., Cole L.A., Serum hCGβ-core fragment is masked by associated macromolecules. J. Clin Endocrinol Metab 1990; 71:1393-1395.
26. Yogeeswaran G., Salk P.L. Metastatic potential is correlated with cell surface sialylation of cultured murine tumor cell lines. Science 1981; 212:1514-1516.
27. Kim U. On the characteristics of tumor cells and host responses associated with metastatic potential. 13th International Cancer Congress. Biology of Cancer(2). Ed. E.A. Mirand, W.B. Hutchinson, E. Mihich. Prog. Clin. Biol. Res. 1983; 132C:45-50.
28. Burchiel S.W., Austin R.K., Rhodes B.A. Expression and detection of hCG in human malignancy. In: Burchiel S.W., Rhodes B.A., Friedman B, eds. The Radio Immunochemical Detection of Cancer: Tumor Imaging. New York: Masson, 1982; 89-97.
29. Raikow, R.B., Acevedo H.F., Krichevsky A, Buffo M.J., Fogarty P. Flow cytofluorometric analyses of choriogonadotropin-like material on the surface of human and mouse malignant cells. Cancer Detect Prev 1987;(Suppl 1) H. Nieburgs & J. Bekes, Ed.:173-181.
30. William C.L., Stewart C.C. General principles of multiparameter flow cytometric analysis: Applications of flow cytometry in the diagnostic pathology laboratory. Semin Diagn Pathol 1989; 6:3-12.
31. American Type Culture Collection. Catalogue of Cell Lines and Hybridomas, 6th Ed. 1988.
32. Patillo, R.A., Gey G.O. Delfs E, Mattingly R.F. Human hormone production in vitro. Science 1968; 159:1467-1469.
33. Patillo, R.A., Gey G.O. The establishment of a cell line of human hormone synthesizing trophoblastic cells in vitro. Cancer Res 1968; 28:1231-1236.
34. Kohler P.O., Bridson W.E. Isolation of hormone-producing clonal lines of human choriocarcinoma. J. Clin Endocrinol 1971; 32:683-687.
35. Ghosh N.K., Cox R.P. Production of human chorionic gonadotropin in HeLa cell cultures. Nature 1976; 259:416-417.
36. Chou J.Y. Regulation on the synthesis of human chorionic gonadotropin by strains of HeLa cells in culture. In Vitro 1978; 14:775-778.
37. Cosgrove D.E., Campain J.A., Cox G.S. Chorionic gonadotropin synthesis by human tumor cell lines. Examination of subunit accumulation, steady-state levels of mRNA, and gene structure. Biochim Biophys Acta 1989; 1007:44-54.
38. Erlich P.H., Moustafa Z.A., Krichevsky A., Birken S., Armstrong E.G., Canfield R.E. Characterization and relative orientation of epitopes for monoclonal antibodies and antisera to human chorionic gonadotropin. Am J. Reprod Immunol Microbiol 1985; 8:48-54.
39. Birken S., Krichevsky A, O'Connor J.O. et al. Development and characterization of monoclonal antibodies to the beta COOH-terminal portion of human chorionic gonadotropin (Abstr 638). Presented at the 72nd Annual Meeting of the Endocrine Society, Atlanta, Georgia, June 20-23, 1990.
40. Stevens V.C., Cinader B, Powell J.E., Lee A.C., Koh S.W. Preparation and formulation of a human chorionic gonadotropin antifertility vaccine: Selection of a peptide immunogen. Am J. Reprod Immunol Microbiol 1981; 1:307-314.
41. Stevens V.C., Cinader B, Powell J.E., Lee A.C., Koh S.W. Preparation and formulation of a human chorionic gonadotropin antifertility vaccine: Selection of adjuvant and vehicle. Am Reprod Immunol Microbiol 1981; 1:315-321.
42. Jones W.R., Judd S, Ing RMY et al. Phase I clinical trial of a World Health Organization birth control vaccine. Lancet 1988; 1:1295-1298.
43. Edwards B.S., Shopp G.M. Efficient use of monoclonal antibodies for immunofluorescence. Cytometry 1989; 10:94-97.
44. Hussa R.O., The Clinical Marker hCG. New York: Praeger, 1987:3-7.
45. Rubin H., Xu K. Evidence for the progressive and adaptive nature of spontaneous transformation of the NIH 3T3 cell line. Proc Natl Acad Sci USA 1989; 86:1860-1864.
46. Edwards B.S., Altobelli K.K., Nolla H.A., Harper D.A., Hoffman R.R. Comprehensive quality assessment approach for flow cytometric immunophenotyping of human lymphocytes. Cytometry 1989; 10:433-441.
47. Darnell R.B., Boime I. Differential expression of the human gonadotropin α gene in ectopic and eutopic cells. Mol Cell Biol 1985; 5:3157-3167.
48. Bunn P.A. Jr., Linnoila I, Minna J.D. Carney D, Gazdar AF. Small cell lung cancer, endocrine cells of the fetal bronchus, and other neuroendocrine cells express the Leu-7 antigenic determinant present on natural killer cells. Blood 1985; 65:764-768.
49. Schulze-Gahmen U., Wilson I.A. Monoclonal antibodies against an identical short peptide sequence shared by two unrelated proteins. Peptide Research 1989; 2:322-331.
50. Cox GS, Rimerman RA. Purification and characterization of the glycoprotein hormone α-subunit-like material secredted by HeLa cells. Biochemistry 1988; 27:6474-6487.
51. Acevedo HF, Campbell-Acevedo EA, Galyon J. Buffo MJ. Expression of membrane-associated human choriogonadotropin and its subunits by cultured human cancer cells (Abstr 168), Presented at the 72nd Annual Meeting of the Endocrine Society, Atlanta, GA, June 20-23, 1990.
52. Kleinbaum, D.G., Kupper, L.L. Applied Regression Analysis and Other Multivariable Methods. Belmont, CA: Duxbury 1978; 83-94 and 271-275.
53. Patillo, R.A., Hussa, R.O., Story, M.T., Ruchert, A.C.F., Shalaby, M.R., Mattingly, R.F. Tumor antigen and human chorionic gonadotrophin in CaSki cells: A new epidermoid cervical cancer cell line. Science (1977) 196:1456-1458.
54. Cowley, G., Smith, J.A., Ellisin, M., Gusterson, B. Production of β-human chorionic gonadotropin by human squamous carcinoma cell lines. Int. J. Cancer (1985) 35:575-579.
55. Iles, R.K., Oliver, R.T.D., Kitau, M., Walker, C., Chart, T. In vitro secretion of human chorionic gonadotropin by bladder tumor cells. Br. J. Cancer (1987) 55:623-626.
56. Ruddon, R.W., Anderson, C., Meade-Coburn, K.S. Stimulation of synthesis and secretion of chorionic gonadotropin subunits by eutopic and ectopic hormone-producing human cell lines. Cancer Res. (1980) 40:4519-4523.
57. Rosen, S.W., Weinstraub, B.C., Aaronson, S.A. Nonrandom ectopic protein production by malignant cells: Direct evidence in vitro. J. Clin. Endocrinol. Metab. (1980) 50:834-841.
58. Bradford, M., A rapid and sensitive method for quantitation of microgram quantities of protein utilizing the principle of protein dye binding. Anal Biochem (1976); 72:248-254.
59. Acevedo, H., Krichevsky, A., Campbell-Acevedo, E., Gaylon, J.C., Buffo, M.J. and Hartstock, R.J., Flow cytometry method for the analysis of membrane-associated human chorionic gonadotropin on human cancer cells. Cancer (1992) 69:1818.
60. Acevedo, H., Krichevsky, A., Campbell-Acevedo, E., Gaylon, J.C., Buffo, M.J. and Hartstock, R.J., Expression of membrane-associated human chorionic gonadotropin, its subunits, and fragments by cultured human cancer cells. Cancer (1992) 69:1829.

## Claims

1. Antibody, specifically reactive with an epitope present on the beta subunit of human chorionic gonadotropin for use in the treatment of patients suffering from a malignant form of cancer.

2. Antibody for the use as claimed in claim 1, which antibody is a monoclonal antibody designated B201 and/or a monoclonal antibody designated B204.

3. Use of an antibody, specifically reactive with an epitope present on the beta subunit of human chorionic gonadotropin for the preparation of a medicament for the treatment of patients suffering from a malignant form of cancer.

4. Use as claimed in claim 3, wherein the antibody is a monoclonal antibody designated B201 and/or a monoclonal antibody designated B204.

5. Antibody recognizing an epitope present on the carboxy-terminal portion (CTP) of the beta subunit of human chorionic gonadotropin for use in the treatment of patients suffering from a malignant form of cancer.

6. Antibody for the use as claimed in claim 5, which antibody is a monoclonal antibody designated CTP-103 and/or a monoclonal antibody designated AS-11.

7. Use of an antibody recognizing an epitope present on the carboxy-terminal portion (CTP) of the beta subunit of human chorionic gonadotropin for the preparation of a medicament for the treatment of patients suffering from a malignant form of cancer.

8. Use as claimed in claim 7, wherein the antibody is a monoclonal antibody designated CTP-103 and/or a monoclonal antibody designated AS-11.

9. A method of lysing malignant cells in vitro, which comprises contacting the malignant cells with an antibody which specifically reacts with an epitope present on the beta subunit of human chorionic gonadotropin and/or beta subunit fragment.

10. The method of claim 9, wherein the antibody is a monoclonal antibody designated B201 and/or a monoclonal antibody designated B204.

## Patentansprüche

1. Antikörper, der spezifisch reaktiv mit einem Epitop ist, das auf der Beta-Untereinheit des menschlichen Choriongonadotropins vorhanden ist, zur Verwendung in der Behandlung von Patienten, die an einer malignen Form von Krebs leiden.

2. Antikörper für die Verwendung nach Anspruch 1, wobei der Antikörper ein monoklonaler Antikörper, der mit B201 bezeichnet wird, und/oder ein monoklonaler Antikörper, der mit B204 bezeichnet wird, ist.

3. Verwendung eines Antikörpers, der spezifisch reaktiv mit einem Epitop ist, das auf der Beta-Untereinheit des menschlichen Choriongonadotropins vorhanden ist, zur Herstellung eines Medikaments für die Behandlung von Patienten, die an einer malignen Form von Krebs leiden.

4. Verwendung nach Anspruch 3, bei der der Antikörper ein monoklonaler Antikörper, der mit B201 bezeichnet wird, und/oder ein monoklonaler Antikörper, der mit B204 bezeichnet wird, ist.

5. Antikörper, der ein Epitop erkennt, das auf dem Carboxyl-Endabschnitt (CTP) der Beta-Untereinheit des menschlichen Choriongonadotropins vorhanden ist, zur Verwendung in der Behandlung von Patienten, die an einer malignen Form von Krebs leiden.

6. Antikörper für die Verwendung nach Anspruch 5, wobei der Antikörper ein monoklonaler Antikörper, der mit CTP-103 bezeichnet wird, und/oder ein monoklonaler Antikörper, der mit AS-11 bezeichnet wird, ist.

7. Verwendung eines Antikörpers, der ein Epitop erkennt, das auf dem Carboxyl-Endabschnitt (CTP) der Beta-Untereinheit des menschlichen Choriongonadotropins vorhanden ist, zur Herstellung eines Medikaments für die Behandlung von Patienten, die an einer malignen Form von Krebs leiden.

8. Verwendung nach Anspruch 7, bei der Antikörper ein monoklonaler Antikörper, der mit CTP-103 bezeichnet wird, und/oder ein monoklonaler Antikörper, der mit AS-11 bezeichnet wird, ist.

9. Verfahren des Lysierens von malignen Zellen in vitro, welches ein In-Kontakt-Bringen der malignen Zellen mit einem Antikörper, der spezifisch mit einem Epitop reagiert, das auf der Beta-Untereinheit des menschlichen Choriongonadotropins und/oder einem Beta-Untereinheits-Fragment vorhanden ist, aufweist.

10. Verfahren nach Anspruch 9, bei dem der Antikörper ein monoklonaler Antikörper, der mit B201 bezeichnet wird, und/oder ein monoklonaler Antikörper, der mit B204 bezeichnet wird, ist.

## Revendications

1. Anticorps, présentant une réactivité spécifique vis-à-vis d'un épitope présent sur la sous-unité bêta de la gonadotropine chorionique humaine, pour utilisation dans le traitement de patients souffrant d'une forme maligne de cancer.

2. Anticorps pour l'utilisation selon la revendication 1, lequel anticorps est un anticorps monoclonal portant la désignation B201 et/ou un anticorps monoclonal portant la désignation B204.

3. Utilisation d'un anticorps présentant une réactivité spécifique vis-à-vis d'un épitope présent sur la sous-unité bêta de la gonadotropine chorionique humaine, pour la préparation d'un médicament destiné au traitement de patients souffrant d'une forme maligne de cancer.

4. Utilisation selon la revendication 3, pour laquelle l'anticorps est un anticorps monoclonal portant la désignation B201 et/ou un anticorps monoclonal portant la désignation B204.

5. Anticorps reconnaissant un épitope présent sur la portion carboxy-terminale (CTP) de la sous-unité bêta de la gonadotropine chorionique humaine, pour utilisation dans le traitement de patients souffrant d'une forme maligne de cancer.

6. Anticorps pour l'utilisation selon la revendication 5, lequel anticorps est un anticorps monoclonal portant la désignation CTP-103 et/ou un anticorps monoclonal portant la désignation AS-11.

7. Utilisation d'un anticorps reconnaissant un épitope présent sur la portion carboxy-terminale (CTP) de la sous-unité bêta de la gonadotropine chorionique humaine, pour la préparation d'un médicament destiné au traitement de patients souffrant d'une forme maligne de cancer.

8. Utilisation selon la revendication 7, pour laquelle l'anticorps est un anticorps monoclonal portant la désignation CTP-103 et/ou un anticorps monoclonal portant la désignation AS-11.

9. Procédé de lyse de cellules malignes in vitro, qui comprend la mise en contact des cellules malignes avec un anticorps qui réagit d'une manière spécifique avec un épitope présent sur la sous-unité bêta de la gonadotropine chorionique humaine et/ou un fragment de la sous-unité bêta.

10. Procédé selon la revendication 9, dans lequel l'anticorps est un anticorps monoclonal portant la désignation B201 et/ou un anticorps monoclonal portant la désignation B204.
